Europäisches Patentamt
European Patent Office
Office européen des brevets

(19)

(11) Veröffentlichungsnummer: **0 233 155 A1**

# (12) EUROPÄISCHE PATENTANMELDUNG

(21) Anmeldenummer: **87810081.7**

(22) Anmeldetag: **10.02.87**

(51) Int. Cl.4: **C 07 D 499/00**
A 61 K 31/43
// C07D205/08, C07F9/65

(30) Priorität: **14.02.86 CH 597/86**

(43) Veröffentlichungstag der Anmeldung:
**19.08.87 Patentblatt 87/34**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Lang, Marc, Dr.**
**Rue des Ormes 6**
**F-68170 Rixheim (FR)**

(54) **Aminoacyloxymethyl-Verbindungen.**

(57) Verbindungen der Formel

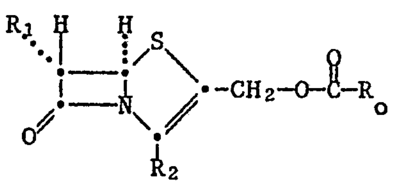

(I),

worin $R_1$ durch Hydroxy oder geschütztes Hydroxy substituiertes Niederalkyl ist, $R_2$ Carboxyl oder funktionell abgewandeltes Carboxyl bedeutet, $R_o$ Azacycloalkyl oder eine Gruppe der Formel $-Y-N(R_3,R_4)$ ist, worin $R_3$ Wasserstoff oder Niederalkyl ist, $R_4$ Wasserstoff, Niederalkyl oder Acyl ist und Y Niederalkylen, durch Phenyl substituiertes Niederalkylen oder ein Rest der Formel $-CR_5R_6-$ ist, worin $R_5$ für einen über ein Kohlenstoffatom gebundenen organischen Rest steht oder zusammen mit $R_3$ gegebenenfalls durch Oxo oder Hydroxy substituiertes Niederalkylen bedeutet, und $R_6$ Wasserstoff oder Niederalkyl bedeutet, die reinen optischen Isomere von Verbindungen der Formel (I) und Mischungen dieser optischen Isomere, und Salze solcher Verbindungen besitzen antibiotische Eigenschaften. Die Verbindungen können nach an sich bekannten Verfahren hergestellt werden.

EP 0 233 155 A1

**Beschreibung**

Aminoacyloxymethyl-Verbindungen

Die Erfindung betrifft 2-aminoacyloxymethyl-2-penem-Verbindungen der Formel

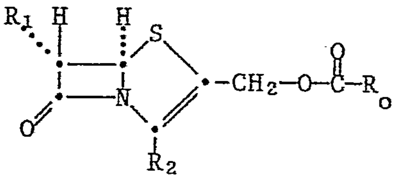

(I),

worin $R_1$ durch Hydroxy oder geschütztes Hydroxy substituiertes Niederalkyl ist, $R_2$ Carboxyl oder funktionell abgewandeltes Carboxyl bedeutet, $R_o$ Azacycloalkyl oder eine Gruppe der Formel $-Y-N(R_3,R_4)$ ist, worin $R_3$ Wasserstoff oder Niederalkyl ist, $R_4$ Wasserstoff, Niederalkyl oder Acyl ist und Y Niederalkylen, durch Phenyl substituiertes Niederalkylen oder ein Rest der Formel $-CR_5R_6-$ ist, worin $R_5$ für einen über ein Kohlenstoffatom gebundenen organischen Rest steht oder zusammen mit $R_3$ gegebenenfalls durch Oxo oder Hydroxy substituiertes Niederalkylen bedeutet und $R_6$ Wasserstoff oder Niederalkyl bedeutet, die reinen optischen Isomere von Verbindungen der Formel (I) und Mischungen dieser optischen Isomere, Salze von Verbindungen der Formel (I), die eine salzbildende Gruppe aufweisen, Verfahren zur Herstellung von Verbindungen der Formel (I), pharmazeutische Präparate, die solche Verbindungen enthalten, und ihre Verwendung zur Herstellung von pharmazeutishcen Präparaten oder als pharmakologisch wirksame Verbindungen.

Die vor- und nachstehend verwendeten Definitionen haben im Rahmen der vorliegenden Beschreibung vorzugsweise die folgenden Bedeutungen:

Funktionell abgewandeltes Carboxyl $R_2$ ist insbesondere unter physiologischen Bedingungen spaltbares verestertes Carboxyl oder geschütztes Carboxyl $R_2'$.

Eine unter physiologischen Bedingungen spaltbare (d.h. metabolisierbare) veresterte Carboxylgruppe $R_2$ ist in erster Linie eine Acyloxymethoxycarbonylgruppe oder auch Acylaminomethoxycarbonylgruppe, worin Acyl z.B. der Rest einer organischen Carbonsäure, in erster Linie einer gegebenenfalls, z.B. durch Amino, substituierten Niederalkancarbonsäure oder Arencarbonsäure, z.B. Benzoesäure, ist oder worin Acyloxymethyl den Rest eines Lactons bildet. Solche Gruppen sind z.B. Niederalkanoyloxymethoxycarbonyl, Aminoniederalkanoyloxymethoxycarbonyl, insbesondere α-Aminoniederalkanoyloxymethoxycarbonyl, und Niederalkanoylaminomethoxycarbonyl. Weitere unter physiologischen Bedingungen spaltbare veresterte Carboxylgruppen $R_2$ sind z.B. 3-Phthalidyloxycarbonyl, 1-Niederalkoxycarbonyloxyniederalkoxycarbonyl, 1-Niederalkoxyniederalkoxycarbonyl oder 2-Oxo-1,3-dioxolen-4-ylmethoxycarbonyl, welches in 5-Stellung des Dioxolenringes gegebenenfalls durch Niederalkyl oder Phenyl substituiert ist.

Eine Acylgruppe $R_4$ hat bis zu 15 Kohlenstoffatome, insbesondere bis zu 7 Kohlenstoffatome, und ist z.B. die Acylgruppe einer Carbonsäure, eines Halbesters der Kohlensäure, einer Carbaminsäure, einer substituierten Carbaminsäure, einer Sulfonsäure, der Amidosulfonsäure oder einer substituierten Amidosulfonsäure. Solche Reste sind beispielsweise Niederalkanoyl, durch Hydroxy, Niederalkoxy, Amino, Halogen, Carboxy und/oder Cyano substituiertes Niederalkanoyl, Benzoyl, durch Amino, Niederalkylamino, Diniederalkylamino, Niederalkanoylamino, Carboxy, Cyano, Nitro, Halogen und/oder Niederalkyl substituiertes Benzoyl, Phenylniederalkanoyl, im Niederalkanoylteil durch Hydroxy oder Amino substituiertes Phenylniederalkanoyl, Carbamoyl oder Sulfo. Als Acylreste $R_4$ kommen auch die bei den Aminoschutzgruppen genannten Acylreste, beispielsweise Niederalkoxycarbonyl, in Betracht.

Ein über Kohlenstoffatom gebundener organischer Rest $R_5$ hat bis zu 18, bevorzugt bis zu 10 Kohlenstoffatome und ist z.B. ein entsprechender gesättigter oder ungesättigter niederaliphatischer, gesättigter oder ungesättigter cycloaliphatischer, aromatischer oder aromatisch-niederaliphatischer Kohlenwasserstoffrest oder ein heterocyclischer oder heterocyclisch-niederaliphatischer Rest.

Ein entsprechender niederaliphatischer Rest $R_5$ ist z.B. Niederalkyl, ferner auch Niederalkenyl.

Ein entsprechender cycloaliphatischer Rest $R_5$ ist beispielsweise Cycloalkyl oder ungesättigtes Cycloalkyl, wie Cycloalkenyl oder Cycloalkadienyl.

Als aromatischer Kohlenwasserstoffrest ist $R_5$ beispielsweise ein entsprechender monocyclischer oder auch polycyclischer, wie bicyclischer Rest, insbesondere Phenyl.

Bei einem aromatisch-niederaliphatischen Kohlenwasserstoffrest $R_5$ handelt es sich beispielsweise um Phenylniederalkyl.

Ein heterocyclischer Rest $R_5$ ist insbesondere ein entsprechender monocyclischer oder polycyclischer, insbesondere monocyclischer oder bicyclischer Rest, wie ein gegebenenfalls partiell gesättigter monocyclischer 5- oder 6-gliedriger Heteroaryl-Rest mit 1-4 Ringstickstoffatomen und gegebenenfalls einem zusätzlichen Ringheteroatom ausgewählt aus der Gruppe Sauerstoff und Schwefel, z.B. ein entsprechender aza-, diaza-, triaza-, tetraza-, oxa-, oxaza-, oxadiaza-, oxatriaza-, thia-, thiaza-, thiadiaza- oder thiatriazacyclischer Rest aromatischen Charakters, oder ein entsprechender Dihydro- oder Tetrahydro-Rest, ferner gegebenenfalls partiell gesättigte Benzo-, Pyrido- oder Pyrimidoderivate solcher 5- oder 6-gliedrigen Reste.

Entsprechende Heteroarylreste $R_5$ sind beispielsweise Pyrrolyl, wie 2- oder 3-Pyrrolyl, Diazolyl, wie Imidazolyl, z.B. 2- oder 4-Imidazoyl, oder Pyrazolyl, z.B. 3-oder 4-Pyrazolyl, Triazolyl, z.B. 1,2,3-Triazol-4-yl oder 1,2,4-Triazol-3-yl, Tetra zolyl, z.B. 1H- oder 2H-Tetrazol-5-yl, Pyridyl, z.B. 2-, 3- oder 4-Pyridyl, Diazinyl, wie Pyrimidyl, z.B. 2-, 4- oder 5-Pyrimidyl, Furyl, z.B. 2- oder 3-Furyl. Thienyl, z.B. 2- oder 3-Thienyl, Oxazolyl, z.B. 2- oder 4-Oxazolyl, Oxadiazolyl, z.B. 1,2,4-Oxadiazol-3-yl oder 1,3,4-Oxadiazol-2-yl, Isoxazolyl, z.B. 3-Isoxazolyl, Thiazolyl, z.B. 2- oder 4-Thiazolyl, Thiadiazolyl, z.B. 1,2,4-Thiadiazol-3-yl oder 1,3,4-Thiadiazol-2-yl, oder Isothiazoyl, z.B. 3- oder 4-Isothiazolyl. Die genannten 5- und 6-gliedrigen Heteroarylreste können auch in partiell gesättigter Form vorliegen. Polycyclische Derivate der genannten Heteroarylreste sind z.B. Indol-3-yl oder Benzimidazol-2-yl.

Heterocyclisch-niederaliphatische Reste $R_5$ sind z.B. Niederalkylreste, die durch einen oder zwei, insbesondere einen, der oben genannten über ein Kohlenstoffatomen gebundenen Heterocyclylreste oder durch einen gegebenenfalls partiell gesättigten monocyclischen 5-oder 6-gliedrigen über ein Ringstickstoffatom gebundenen Heteroarylrest, welcher als Ringheteroatome 1-4 Stickstoffatome enthält, substituiert ist. Bei den zuletzt genannten über ein Stickstoffatom gebundenen Resten handelt es sich beispielsweise um Imidazol-1-yl, Pyrazol-1-yl, 1,2,4-Triazol-1-yl, Tetrazol-1-yl, Tetrazol-2-yl oder 1-Pyridinio.

Die genannten organischen Reste $R_5$ sind unsubstituiert oder können z.B. durch gegebenenfalls veräthertes oder verestertes, inklusive geschütztes Hydroxy, z.B. Hydroxy, Niederalkoxy, Niederalkanoyloxy oder Halogen, gegebenenfalls veräthertes Mercapto, z.B. Mercapto oder Niederalkylthio, Niederalkyl; durch Hydroxy, Niederalkoxy, gegebenenfalls verestertes oder amidiertes Carboxy, wie Carboxy, Niederalkoxycarbonyl oder Carbamoyl, gegebenenfalls N-niederalkyliertes Amino, z.B. Amino, Niederalkylamino oder Diniederalkylamino, Niederalkylenamino, Niederalkanoylamino, gegebenenfalls durch Amino und/oder Carboxy substituiertes Niederalkoxycarbonylamino, Mercapto, Niederalkylthio oder Sulfo substituiertes Niederalkyl; gegebenenfalls substituiertes Amino, z.B. Amino, Niederalkylamino, Diniederalkylamino, Niederalkylamino, Guanidino, Ureido, Acylamino, wie Niederalkanoylamino, oder gegebenenfalls durch Amino und/oder Carboxy substituiertes Niederalkoxycarbonylamino; gegebenenfalls funktionell abgewandeltes Carboxyl oder Sulfo, z.B. Carboxyl, verestertes Carboxyl, wie Niederalkoxycarbonyl, amidiertes Carboxyl, wie gegebenenfalls substituiertes Carbamoyl, z.B. Carbamoyl oder N-mono- oder N,N-diniederalkyliertes Carbamoyl, Cyano, Sulfo oder Sulfamoyl, gegebenenfalls z.B. durch Niederalkyl, Nitro, Amino, Hydroxy, Niederalkoxy, Carboxy und/oder Halogen substituiertes Phenyl, Nitro, Oxo und/oder Oxido substituiert, wie z.B. mono- oder auch poly-, wie insbesondere mono- oder di-, ferner auch trisubstituiert sein.

In der vorliegenden Beschreibung bedeutet der im Zusammenhang mit Definitionen von Gruppen bzw. Verbindungen verwendete Ausdruck "Nieder", das die entsprechenden Gruppen bzw. Verbindungen, sofern nicht ausdrücklich anders definiert, 1 bis 7, bevorzugt 1 bis 4 Kohlenstoffatome enthalten.

Durch Hydroxy substituiertes Niederalkyl $R_1$ ist insebsondere in $\alpha$-Stellung zum Penem-Ringgerüst durch Hydroxy substituiertes Niederalkyl und bedeutet z.B. 1-Hydroxyprop-1-yl, 2-Hydroxyprop-2-yl oder 1-Hydroxybut-1-yl und insbesondere Hydroxymethyl oder 1-Hydroxyäthyl.

Niederalkanoyloxymethoxycarbonyl ist z.B. Acetoxymethoxycarbonyl oder Pivaloyloxymethoxycarbonyl.

$\alpha$-Aminoniederalkanoyloxymethoxycarbonyl ist z.B. Glycyloxymethoxycarbonyl, L-Valyloxymethoxycarbonyl oder L-Leucyloxymethoxycarbonyl.

Niederalkanoylaminomethoxycarbonyl ist z.B. Acetaminomethoxycarbonyl.

1-Niederalkoxycarbonyloxyniederalkoxycarbonyl ist z.B. Aethoxycarbonyloxymethoxycarbonyl oder 1-Aethoxycarbonyloxyäthoxycarbonyl.

1-Niederalkoxyniederalkoxycarbonyl ist z.B. Methoxymethoxycarbonyl oder 1-Methoxyäthoxycarbonyl.

Bei einer 2-Oxo-1,3-dioxolen-4-ylmethoxy-Gruppe, welche, in 5-Stellung des Dioxolen-Ringes gegebenenfalls durch Niederalkyl oder Phenyl substituiert ist, handelt es sich vor allem um eine 5-Phenyl-und in erster Linie um eine 5-Methyl-2-oxo-1,3-dioxolen-4-ylmethoxy-Gruppe.

Niederalkyl ist z.B. n-Propyl, Isopropyl, n-Butyl, Isobutyl, sek.-Butyl oder tert.-Butyl, ferner auch n-Pentyl, n-Hexyl oder n-Heptyl, in erster Linie jedoch Methyl oder Aethyl.

Azacycloalkyl $R_o$ hat z.B. 3 bis 8, insbesondere 5 oder 6, Ringglieder und ist über das Ringstickstoffatom oder insbesondere über ein Ringkohlenstoffatom an die Carbonylgruppe -C(=O)- gebunden. Solche Reste sind beispielsweise Azetidinyl, z.B. Azetidin-2-yl, Pyrrolidinyl, z.B. Pyrrolidin-2-yl oder Pyrrolidin-1-yl, und Piperidinyl, z.B. 2-, 3- oder 4-Piperidinyl.

Niederalkanoyl ist z.B. Formyl, Acetyl oder Propionyl, während Phenylniederalkanoyl z.B. Phenylacetyl ist.

Niederalkoxy ist z.B. Methoxy, ferner Aethoxy, n-Propyloxy, Isopropyloxy, n-Butyloxy, Isobutyloxy oder tert.-Butyloxy, sowie n-Pentyloxy, n-Hexyloxy oder n-Heptyloxy.

Halogen ist z.B. Fluor, Chlor, Brom oder Jod.

Niederalkylamino ist z.B. Methylamino, Aethylamino, n-Propylamino, Isopropylamino oder n-Butylamino, während Diniederalkylamino z.B. Dimethylamino, Diäthylamino, Di-n-propylamino oder Diisopropylamino bedeutet.

Niederalkanoylamino ist z.B. Formylamino, Acetylamino oder Propionylamino.

Niederalkylen Y ist geradkettiges Niederalkylen mit 1 bis 12 Kohlenstoffatomen, insbesondere mit 1 bis 7 Kohlenstoffatomen, wie Methylen, 1,2-Aethylen, 1,3-Propylen, 1,4-Butylen, 1,5-Pentylen, 1,6-Hexylen, 1,7-Heptylen, 1,8-Octylen oder 1,10-Decylen, oder verzweigtkettiges Niederalkylen mit 2 bis 10 Kohlenstoffatomen, insbesondere mit 2 bis 7 Kohlenstoffatomen, wie durch Niederalkyl, z.B. Methyl oder Aethyl, mono- oder disubstituiertes geradkettiges Niederalkylen, z.B. Aethyliden, 1,2-Propylen, 1,2-Butylen, 1,3-Butylen,

2-Methyl-1,2-propylen, 3-Methyl-1,3-butylen oder 2-Methyl-2,3-butylen, wobei das Kohlenstoffatom 1 der genannten Reste der Carbonylgruppe -C(=O)- oder der Aminogruppe -N($R_3$,$R_4$) benachbart sein kann.

Durch Phenyl substituiertes Niederalkylen Y hat im Niederalkylenteil 2 bis 7 Kohlenstoffatome und ist z.B. 1-Phenyl-1,2-äthylen oder 1-Phenyl-1,3-propylen, wobei das Kohlenstoffatom 1 der genannten reste der Carbonylgruppe -C(=O)- oder Aminogruppe -N($R_3$,$R_4$) benachbart sein kann.

Niederalkenyl hat 2 bis 7 Kohlenstoffatome und ist z.B. Vinyl, Allyl, 2-Methylallyl, n-Propenyl oder Isopropenyl.

Cycloalkyl enthält vorzugweise 3 bis 8, in erster Linie 5 oder 6 Ringglieder und ist zum Beispiel Cyclopentyl oder Cyclohexyl, ferner Cyclopropyl sowie Cycloheptyl, während Cycloalkenyl z.B. 5 oder 6 Ringglieder und Cycloalkadienyl z.B. 6 Ringglieder enthält und z.B. 1-, 2-oder 3-Cyclohexenyl, 1- oder 2-Cyclopentenyl oder 1,4-Cyclohexadienyl ist.

Phenylniederalkyl ist z.B. Benzyl, 1-Phenyläthyl oder 2-Phenyläthyl.

Nideralkanoyloxy ist z.B. Formyloxy, Acetyloxy oder Propionyloxy.

Niederalkylthio ist z.B. Methylthio oder Aethylthio, ferner auch n-Propylthio, Isopropylthio oder n-Butylthio.

Niederalkoxycarbonyl ist z.B. Methoxycarbonyl oder Aethoxycarbonyl.

Niederalkylenamino weist insbesondere 4 bis 6 Kohlenstoffatome auf und ist z.B. Pyrrolidino oder Piperidino.

Gegebenenfalls durch Amino und/oder Carboxy substituiertes Niederalkoxycarbonylamino ist z.B. Methoxycarbonylamino, Aethoxycarbonylamino oder Isopropoxycarbonylamino, ferner 2-Amino-2-carboxy-äthoxycarbonylamino.

N-Mono-niederalkyliertes Carbamoyl ist z.B. N-Methyl-, N-Aethyl-oder N-Propylcarbamoyl, während N,N-Di-niederalkyliertes Carbamoyl z.B. N,N-Dimethyl- oder N,N-Diäthylcarbamoyl bedeutet.

Eine gemeinsam von den Resten $R_3$ und $R_5$ gebildete, gegebenenfalls durch Hydroxy oder Oxo substituierte Niederalkylen-Gruppe hat 2 bis 5 Kohlenstoffatome und ist insbesondere 1,3-Propylen, 2-Hydroxy-1,3-propylen oder 1-Oxo-1,3-propylen, worin das Kohlenstoffatom 1 der Propylen-Gruppen dem Rest -$\overset{|}{N}$-$R_4$ benachbart ist.

Beispiele für niederaliphatische Reste $R_5$ sind z.B. Niederalkyl, z.B. Methyl, Aethyl, Isopropyl, Isobutyl oder sek.-Butyl, oder durch Hydroxy, Niederalkoxy, z.B. Methoxy oder Aethoxy, Niederalkanoyloxy, z.B. Acetyloxy, Halogen, z.B. Chlor oder Brom, Mercapto, Niederalkylthio, z.B. Methylthio, Amino, Niederalkylamino, z.B. Methyl-oder Aethylamino, Diniederalkylamino, z.B. Dimethylamino, Guanidino, Ureido, Niederalkanoylamino, z.B. Acetylamino, durch Amino und Carboxy substituiertes Niederalkoxycarbonylamino, wie Aethoxycarbonylamino, z.B. 2-Amino-2-carboxyäthoxycarbonylamino, Carboxyl, Niederalkoxycarbonyl, z.B. Methoxy- oder Aethoxycarbonyl, Carbamoyl, Sulfo, Sulfamoyl und/oder Oxo substituiertes Niederalkyl, wie insbesondere Methyl, Aethyl, n-Propyl oder n-Butyl. Repräsentative Vertreter solcher niederaliphatischer Reste $R_5$ sind z.B. Methyl, Isopropyl, Isobutyl, sek.-Butyl, Hydroxymethyl, 1- oder 2-Hydroxyäthyl, Mercaptomethyl, 2-Methylthioäthyl, Aminomethyl, 1- oder 2-Aminoäthyl, 3-Aminopropyl, 4-Aminobutyl, 3-Guanidylpropyl, 3-Ureidopropyl, Carboxymethyl, Carbamoylmethyl, 2-Carboxyäthyl, 2-Carbamoyläthyl, Carboxyl oder Niederalkoxycarbonyl, wie Methoxycarbonyl.

Beispiele für cycloaliphatische Reste $R_5$ sind z.B. Cycloalkenyl, z.B. 1-oder 2-Cyclohexenyl, oder insbesondere Cyclohexadienyl, z.B. 1,4-Cyclohexadienyl.

Aromatische Kohlenwasserstoffreste $R_5$ sind z.B. Phenyl oder durch Amino, Niederalkylamino, z.B. Methyl- oder Aethylamino, Diniederalkylamino, z.B. Dimethylamino, Niederalkyl, z.B. Methyl, Aminoniederalkyl, z.B. 2-Aminoäthyl, Niederalkylaminoniederalkyl, z.B. 2-Methylaminoäthyl, durch Amino und Carboxy substituiertes Niederalkoxycarbonylaminoniederalkyl, z.B. 2-(2-Amino-2-carboxy-äthoxycarbonylamino)-äthyl, durch Amino und Carboxy substituiertes Niederalkoxycarbonylamino, z.B. 2-amino-2-carboxyäthoxycarbonylamino, Nitro, Hydroxy, Niederalkoxy, z.B. Methoxy oder Aethoxy, Halogen, z.B. Chlor oder Brom, oder Carboxy substituiertes, wie insbesondere mono-, aber auch disubstituiertes Phenyl.

Aromatisch-niederaliphatische Reste $R_5$ sind beispielsweise gegebenenfalls substituiertes Phenylniederalkyl, wie insbesondere Benzyl, worin die Substituenten z.B. Niederalkyl, wie Methyl, Nitro, Hydroxy, Niederalkoxy, z.B. Methoxy oder Aethoxy, Carboxy und/oder Halogen, z.B. Chlor, sind.

Heterocyclische Reste $R_5$ sind z.B. gegebenenfalls z.B. durch Carboxy, Niederalkyl, Aminoniederalkyl oder Amino substituiertes Imidazolyl, z.B. 2- oder 4-Imidazolyl oder 2-Amino-4-imidazoyl, gegebenenfalls z.B. durch Niederalkyl, Amino, Carboxyniederalkyl und/oder Phenyl substituiertes Triazolyl, wie 1H-1,2,3-Triazol-4-yl oder 1H-1,2,4-Triazol-3-yl, z.B. die entsprechenden unsubstituierten Reste, 5-Methyl- oder 5-Amino-1H-1,2,4-triazol-3-yl, gegebenenfalls z.B. durch Niederalkyl, Carboxyniederalkyl, Sulfoniederalkyl, Diniederalkylaminoniederalkyl oder gegebenenfalls Substituenten, wie Halogen, enthaltendes Phenyl substituiertes Tetrazolyl, wie 1H- oder 2H-Tetrazol-5-yl, z.B. 1H-Tetrazol-5-yl, 1-Carboxymethyl-, 1-(2-Carboxyäthyl)-, 1-(2-Sulfoäthyl)-, 1-(2-Dimethylaminoäthyl)- oder 1-Phenyl-1H-tetrazol-5-yl, gegebenenfalls z.B. durch Niederalkyl oder Amino substituiertes Thiazolyl, wie 2-oder 4-Thiazolyl, z.B. 2-Thiazolyl, 2-Amino-4-thiazolyl oder 4-5-Dimethyl-2-thiazolyl, gegebenenfalls z.B. durch Amino, Niederalkyl und/oder Phenyl substituiertes Oxazolyl, wie 2- oder 4-Oxazolyl, z.B. die entsprechenden unsubstituierten Gruppen, ferner 4-Methyl-2-oxazolyl oder 2-Amino-4-oxazolyl, gegebenenfalls z.B. durch Halogen, Niederalkyl oder Aminoniederalkyl substituiertes Furyl oder Thienyl, wie 2- oder 3-Furyl oder 2- oder 3-Thienyl, z.B. die entsprechenden unsusbtituierten Reste, 5-Methyl- oder 5-Aminomethyl-2-furyl, oder 5-Aminomethyl-2-thienyl oder gegebenenfalls z.B. durch Hydroxy, Halogen, Niederalkyl, Amino und/oder Oxido substituiertes Pyridyl, wie 2-, 3-oder 4-Pyridyl, z.B. die entsprechenden

unsusbtituierten Gruppen, 1-Oxido-2-pyridyl oder 4-Chlor-1-oxido-2-pyridyl.

Heterocyclisch-niederaliphatische Reste $R_5$ sind z.B. gegebenenfalls substituierte Heterocyclylmethyl-Reste, wie z.B. gegebenenfalls z.B. durch Niederalkyl substituiertes Imidazol-1-ylmethyl oder -4-ylmethyl z.B. Imidazol-4-ylmethyl, oder gegebenenfalls z.B. durch Niederalkyl, Carboxyniederalkyl, Sulfoniederalkyl, Diniederalkylaminoniederalkyl, Amino oder gegebenenfalls durch Halogen substituiertes Phenyl substituiertes Tetrazolylmethyl, wie 1H-Tetrazol-1-ylmethyl, 2H-Tetrazol-2-ylmethyl oder 1H-Tetrazol-5-ylmethyl, z.B. die entsprechenden unsubstituierten Reste, 5-Amino-, 5-Carboxymethyl-, 5-(2-Carboxyäthyl)-, 5-Sulfomethyl-, 5-(2-Dimethylaminoäthyl)- oder 5-Phenyl-1H-tetrazol-1-ylmethyl, 5-Amino-, 5-Carboxymethyl-, 5-Sulfomethyl-, 5-(2-Dimethylaminoäthyl)- oder 5-Phenyl-2H-tetrazol-2-ylmethyl, oder 1-(2-Carboxyäthyl)- oder 1-(2-Dimethylaminoäthyl)-2H-tetrazol-5-ylmethyl, Pyridylmethyl, wie Pyridiniomethyl oder 2-, 3- oder 4-Pyridylmethyl, Furylmethyl, z.B. 2-Furylmethyl, Thienylmethyl, z.B. 2-Thienylmethyl, gegebenenfalls z.B. durch Amino substituiertes Oxazolylamethyl, Thiazolylmethyl oder Thiadiazolylmethyl, z.B. 2-amino-oxazol-4-ylmethyl, 2-Amino-thiazol-4-ylmethyl oder 5-Amino-1,2,4-thiadiazol-3-ylmethyl, oder Indolylmethyl, z.B. Indol-3-ylmethyl.

Bevorzugt als Rest $R_1$ sind Hydroxymethyl und in erster Linie 1-Hydroxyäthyl.

Bevorzugte unter physiologischen Bedingungen spaltbare veresterte Carboxylgruppen $R_2$ sind z.B. Niederalkanoyloxymethoxycarbonyl, z.B. Acetoxymethoxycarbonyl oder Pivaloyloxymethoxycarbonyl, und 1-Niederalkoxycarbonyloxyniederalkoxycarbonyl, z.B. 1-Aethoxy-carbonyloxyäthoxycarbonyl.

In bevorzugten Verbindungen der Formel (I) steht der Rest $R_o$ für den Acylrest einer natürlichen, insbesondere einer genetisch enkodierten Aminosäure, wie z.B. für den Acylrest von Glycin, Alanin, Valin, Leucin, Isoleucin, Prolin, Serin, Threonin, Methionin, Phenylalanin, Tryptophan, Tyrosin, Asparagin, Asparaginsäure, Glutamin, Glutaminsäure, Lysin, Arginin oder Histidin, ferner für den Acylrest einer genetisch nicht kodierten Aminosäure, z.B. einer durch Amino und gegebenenfalls zusätzlich durch Phenyl, Hydroxy und/oder Ureido substituierten Niederalkancarbonsäure mit 3 bis 12 Kohlenstoffatomen oder einer gegebenenfalls durch Oxo oder Hydroxy substituierten Azacyclycarbonsäure mit 5 oder 6 Ringgliedern im Azacyclyl-Ring, z.B. β-Alanin, Homoserin, 2-, 3- oder 4-Aminobuttersäure, 2,3-Diaminopropionsäure, 2,3-Diaminobuttersäure, 5-Aminopentansäure, 6-Aminohexansäure, 8-Aminooctansäure, 11-Aminoundecansäure, α- oder β-Aminoisobuttersäure, 3-Amino-3-methyl-buttersäure, 4-Amino-4-methyl-pentansäure, 4-Amino-1-methyl-buttersäure, Aminopivalinsäure, 4-Aminopentansäure, 3-Amino-3-phenyl-propionsäure, Citrullin, Ornithin, Piperidin-3-yl-carbonsäure, Piperidin-4-yl-carbonsäure, 3-Hydroxyprolin und Pyrrolidin-5-on-2-carbonsäure, sowie N-niederalkylierten Derivaten, wie den N-Methyl-Derivaten davon, z.B. Sarcosin. Solche Acylreste können sowohl in der natürlichen, d.h. L-Konfiguration, als auch in der D-Konfiguration oder als Gemisch beider Konfigurationen (DL-Form) vorliegen.

Die in den Verbindungen der Formel I vorhandenen funktionellen Gruppen, wie Hydroxy-, Carboxy-, Amino- oder Sulfogruppen, insbesondere die Hydroxygruppe im Rest $R_1$ und die Carboxylgruppe $R_2$, sind gegebenenfalls durch Schutzgruppen geschützt, die in der Penem-, Penicillin-, Cephalosporin- und Peptidchemie verwendet werden. Solche Schutzgruppen schützen die betreffenden funktionellen Gruppen vor unerwünschten Kondensationsreaktionen, Substitutionsreaktionen und dergleichen während der Synthese der Verbindung der Formel I aus ihren Vorstufen.

Solche Schutzgruppen sind leicht, d.h. ohne dass unerwündschte Nebenreaktionen stattfinden, beispielsweise solvolytisch, reduktiv oder auch unter physiologischen Bedingungen, abspaltbar.

Schutzgruppen dieser Art sowie ihre Einführung und Abspaltung sind beispielsweise beschrieben in
J.F.W. McOmie, "Protective Groups in Organic Chemistry", Plenum Press, London, New York, 1973,
T.W. Greene, "Protective Groups in Organic Synthesis", Wiley, New York, 1981,
"The Peptides", Vol. I, Schroeder und Luebke, Academic Press, London, New York, 1965 und
Houben-Weyl, "Methoden der Organischen Chemie", Band 15/1, Georg Thieme Verlag, Stuttgart, 1974.

In Verbindungen der Formel (I) kann eine Hydroxygruppe im Rest $R_1$, ferner eine im Rest $R_o$ vorhandene Hydroxygruppe, beispielsweise durch Acylreste geschützt sein. Geeignete Acylreste sind z.B. gegebenenfalls durch Halogen substituiertes Niederalkanoyl, z.B. Acetyl, Dichloracetyl oder Trifluoracetyl, gegebenenfalls durch Nitro substituiertes Benzoyl, z.B. Benzoyl, 4-Nitrobenzoyl oder 2,4-Dinitrobenzoyl, gegebenenfalls durch Halogen substituiertes Niederalkoxycarbonyl, z.B. 2-Bromäthoxycarbonyl oder 2,2,2-Trichloräthoxycarbonyl, Niederalkenyloxycarbonyl, z.B. Allyloxycarbonyl, Niederalkenyloxyoxalyl, z.B. Allyloxyoxalyl, oder gegebenenfalls durch Nitro substituiertes Phenylniederalkoxycarbonyl, z.B. Benzyloxycarbonyl oder 4-Nitrobenzyloxycarbonyl. Weitere geeignete Hydroxyschutzgruppen sind z.B. trisubstituiertes Silyl, wie Triniederalkylsilyl, z.B. Trimethylsilyl, Dimethyl-(2,3-dimethylbutyl)-silyl oder tert.-Butyl-dimethylsilyl. Bevorzugt als Hydroxyschutzgruppe sind Triniederalkylsilyl, Niederalkenyloxyoxalyl und Niederalkenyloxycarbonyl.

Eine Carboxylgruppe $R_2$, ferner auch eine im Rest $R_o$ vorhandene Carboxylgruppe, ist üblicherweise in veresterter Form geschützt, wobei die Estergruppe unter schonenden Bedingungen, z.B. unter schonend reduktiven, wie hydrogenolytischen, oder schonend solvolytischen, wie acidolytischen oder insbesondere basisch oder neutral hydrolytischen Bedingungen, leicht spaltbar ist.

Solche veresterten Carboxylgruppen enthalten als veresternde Gruppen in erster Linie in 1-Stellung verzweigte oder in 1- oder 2-Stellung geeignet substituierte Niederalkylgruppen. Geeignete in veresterter Form vorliegende Carboxylgruppen sind unter anderen gegebenenfalls durch Nitro oder Niederalkoxy, wie Methoxy, substituiertes Benzyloxycarbonyl, z.B. 4-Nitrobenzyloxycarbonyl oder 4-Methoxybenzyloxycarbonyl, Niederalkanoylmethoxycarbonyl, wie Acetonyloxycarbonyl, Benzoylmethoxycarbonyl, worin die Benzoylgruppe vorzugsweise gegebenenfalls, z.B. durch Halogen, wie Brom, substituiert ist, z.B. Phenacyloxycarbonyl,

Halogenniederalkoxycarbonyl, wie 2-Halogenniederalkoxycarbonyl, z.B. 2,2,2,-Trichloräthoxycarbonyl oder 2-Bromäthoxycarbonyl, Niederalkenyloxycarbonyl, z.B. Allyloxycarbonyl, oder in 2-Stellung durch Niederalkylsulfonyl, Cyano oder trisubstituiertes Silyl, wie Triniederalkylsilyl oder Triphenylsilyl, substituiertes Aethoxycarbonyl, z.B. 2-Methylsulfonyläthoxycarbonyl, 2-Cyanoäthoxycarbonyl, 2-Trimethylsilyläthoxycarbonyl oder 2-(Di-n-butyl-methyl-silyl)-äthoxycarbonyl. Bevorzugte geschüzte Carboxylgruppen $R_2'$ sind die 4-Nitrobenzyloxycarbonyl-, Niederalkenyloxycarbonyl-, insbesondere Allyloxycarbonyl-, und die in 2-Stellung durch Triniederalkylsilyl, z.B. Trimethylsilyl oder Di-n-butyl-methylsilyl, substituierte Aethoxycarbonylgruppe.

Eine geschützte Aminogruppe, z.B. im Rest $R_o$, kann beispielsweise in Form einer leicht spaltbaren Acylaminogruppe oder als Azidogruppe vorliegen. In einer entsprechenden Acylaminogruppe ist Acyl beispielsweise der Acylrest einer organischen Säure mit z.B. bis zu 18 Kohlenstoffatom, insbesondere einer gegebenenfalls z.B. durch Halogen oder Phenyl, substituierten Niederalkancarbonsäure oder insbesondere eines Kohlensäurehalbesters. Solche Acylgruppen sind beispielsweise Halogenniederalkanoyl, wie 2-Halogenacetyl, insbesondere 2,2,2-Trifluor- oder 2,2,2-Trichloracetyl, Niederalkenyloxycarbonyl, z.B. Allyloxycarbonyl, gegebenenfalls in 1- oder 2-Stellung substituiertes Niederalkoxycarbonyl, wie Niederalkoxycarbonyl, z.B. Methoxycarbonyl, Aethoxycarbonyl oder tert.Butoxycarbonyl, gegebenenfalls substituiertes Benzyloxycarbonyl, z.B. 4-Nitrobenzyloxycarbonyl, oder 2-Halogenniederalkoxycarbonyl, z.B. 2,2,2-Trichloräthoxycarbonyl oder 2-Bromäthoxycarbonyl, oder 2-(trisubstituiertes Silyl)-äthoxycarbonyl, wie 2-Triniederalkylsilyläthoxycarbonyl, z.B. 2-Trimethylsilyläthoxycarbonyl oder 2-(Di-n-butylmethyl-silyl)-äthoxycarbonyl. Bevorzugte geschtützte Aminogruppen sind z.B. Azido, Niederalkenyloxycarbonylamino, z.B. Allyloxycarbonylamino, und gegebenenfalls durch Nitro substituiertes Benzyloxycarbonylamino.

Salze von erfindungsgemässen Verbindungen sind in erster Linie pharmazeutisch annehmbare, nicht-toxische Salze von Verbindungen der Formel I. Solche Salze werden beispielsweise von den in Verbindungen der Formel I vorhandenen sauren Gruppen, z.B. Carboxylgruppen, gebildet und sind in erster Linie Metal- oder Ammoniumsalze, wie Alkalimetall- und Erdalkalimetall-, z.B. Natrium-, Kalium-, Magnesium- oder Kalziumsalze, sowie Ammoniumsalze mit Ammoniak oder geeigneten organischen Aminen, wie Niederalkylaminen, z.B. Triäthylamin, Hydroxyniederalkylaminen, z.B. 2-Hydroxyäthylamin, Bis-(2-hydroxyäthyl)-amin oder Tris-(2-hydroxyäthyl)-amin, basischen aliphatischen Estern von Carbonsäuren, z.B. 4-Aminobenzoesäure-2-diäthylaminoäthylester, Niederalkylenaminen, z.B. 1-Aethyl-piperidin, Cycloalkylaminen, z.B. Dicyclohexylamin, oder Benzylaminen, z.B. N,N'-Dibenzyläthylendiamin, Dibenzylamin oder N-Benzyl-β-phenäthylamin. Verbindungen der Formel I mit einer basischen Gruppe, z.B. mit einer Aminogruppe, können Säureadditionssalze, z.B. mit anorganischen Säuren, wie Salzsäure, Schwefelsäure oder Phosphorsäure, oder mit geeigneten organischen Carbon- oder Sulfonsäure, z.B. Essigsäure, Bernsteinsäure, Fumarsäure, Maleinsäure, Weinsäure, Oxalsäure, Zitronensäure, Benzoesäure, Mandelsäure, Aepfelsäure, Ascorbinsäure, Methansulfonsäure oder 4-Toluolsulfonsäure bilden. Verbindungen der Formel I mit einer sauren und mit einer basischen Gruppe können auch in Form von inneren Salze, d.h. in zwitterionischer Form, vorliegen.

Zur Isolierung oder Reinigung können auch pharmazeutisch ungeeignete Salze Verwendung finden. Zur therapeutischen Anwendung gelangen nur die pharmazeutisch annehmbaren, nicht-toxischen Salze, die deshalb bevorzugt sind.

Die Penem-Verbindungen der Formel I können in den Resten $R_1$, und/oder $R_o$ zusätzliche Chiralitätszentren besitzen. Beispielsweise kann 1-Hydroxyäthyl als Substituent $R_1$ in der R-, in der S- oder in der racemischen R,S-Konfiguration vorliegen. In bevorzugten Penem-Verbindungen der Formel I weist ein Rest $R_1$, welcher ein asymmetrisches Kohlenstoffatom besitzt, insbesondere 1-Hydroxyäthyl, die R-Konfiguration auf. Ferner kann beispielsweise das Methin-Kohlenstoffatom im Rest $-CR_5R_6-$ eine asymmetrische Substitution aufweisen und dementsprechend in der R-, S- oder der racemischen R,S-Konfiguration vorliegen. Die Erfindung betrifft demgemäss die reinen Diastereomeren und Mischungen der Diastereomeren von Verbindungen der Formel I, welche im Rest $R_1$ und/oder $R_o$ zusätzliche Chiralitätszentren aufweisen.

Die Verbindungen der Formel I weisen wertvolle pharmakologische Eigenschaften auf oder können als Zwischenprodukte zur Herstellung von solchen Verbindungen mit wertvollen pharmakologischen Eigenschaften verwendet werden. Verbindungen der Formel I, worin $R_1$ durch Hydroxy substituiertes Niederalkyl ist, $R_o$ die unter Formel I angegebenen Bedeutungen hat, wobei funktionelle Gruppen in ungeschützter Form vorliegen, und $R_2$ Carboxyl oder eine unter physiologischen Bedingungen spaltbare veresterte Carboxylgruppe darstellt, und pharmakologisch annehmbare Salze von solchen Verbindungen mit salzbildenden Gruppen haben antibakterielle Wirkungen. Beispielsweise sind sie in vitro gegen grampositive und gramnegative Kokken, inklusive Methicillin-resistente Staphylokokken, z.B. Staphylococcus aureus, Streptococcus pyogenes, Streptococcus pneumoniae und Streptococcus faecalis, in minimalen Konzentrationen von ca. 0,05 bis ca. 8 µg/ml und gegen gramnegative Stäbchenbakterien, wie Enterobacteriaceae, Haemophilus influenzae und Pseudomonas aeruginosa, und Anaerobier, z.B. Bacteroides sp., in minimalen Konzentrationen von ca. 0,05 bis ca. 16 µg/ml wirksam. In vivo, bei der systemischen Infektion der Maus, z.B. durch Staphylocuccus aureus, ergeben sich bei subkutaner oder oraler Applikation erfindungsgemässer Verbindungen $ED_{50}$-Werte von ca. 2,5 bis ca. 10 mg/kg.

Die neuen Verbindungen können als oral parenteral applizierbare antibakterielle Antibiotika, z.B. in Form von entsprechenden pharmazeutischen Präparaten, zur Behandlung von Infektionen Verwendung finden.

Verbindungen der Formel I, worin mindestens eine der vorhandenen funktionellen Gruppen in geschützter Form vorliegt, können als Zwischenprodukte zur Herstellung der oben genannten pharmakologisch wirksamen Verbindungen der Formel I verwendet werden.

Die Erfindung betrifft vor allem Verbindungen der Formel I, worin $R_1$ durch Hydroxy oder geschütztes Hydroxy substituiertes Niederalkyl ist; $R_2$ Carboxyl, unter physiologischen Bedingungen spaltbares verestertes Carboxyl oder geschütztes Carboxyl $R_2'$ bedeutet; $R_o$Azacycloalkyl oder eine Gruppe der Formel -Y-N($R_3$,$R_4$) ist, worin $R_3$ Wasserstoff oder Niederalkyl ist; $R_4$ Wasserstoff, Niederalkyl, Niederalkanoyl, durch Hydroxy, Niederalkoxy, Amino, Halogen, Carboxy und/oder Cyano substituiertes Niederalkanoyl; Benzoyl, durch Amino, Niederalkylamino, Diniederalkylamino, Niederalkanoylamino, Carboxy, Halogen, Nitro, Cyano und/oder Niederalkyl substituiertes Benzoyl, Phenylniederalkanoyl, im Niederalkanoylteil durch Amino oder Hydroxy substituiertes Phenylniederalkanoyl, Carbamoyl, Sulfo oder eine Aminoschutzgruppe ist und Y geradkettiges Niederalkylen mit 1 bis 12 Kohlenstoffatomen, verzweigtes Niederalkylen mit 2 bis 10 Kohlenstoffatomen, durch Phenyl substituiertes Niederalkylen oder eine Gruppe der Formel -$CR_5R_6$- ist, worin $R_5$ Niederalkyl, Niederalkenyl, Cycloalkyl, Cycloalkenyl, Cycloalkadienyl, Phenyl, Phenylniederalkyl, einen über ein Ringkohlenstoffatomen gebundenen monocyclischen 5-oder 6-gliedrigen gegebenenfalls partiell gesättigten Heteroarylrest mit 1-4 Ringstickstoffatomen und gegebenenfalls einem zusätzlichen Ringheteroatom ausgewählt aus der Gruppe Sauerstoff und Schwefel, z.B. einen entsprechenden aza-, diaza-, triaza-, tetraza-, oxa-, oxaza-, oxadiaza-, oxatriaza-, thia-, thiaza-, thiadiaza- oder thiatriaza-cyclischen Rest aromatischen Charakters, oder einen entsprechenden Dihydro- oder Tetrahydrorest, oder ein gegebenenfalls partiell gesättigtes Benzo-, Pyrido- oder Pyrimido-Derivat eines solchen 5- oder 6-gliedrigen Restes, oder einen durch einen über ein Ringkohlenstoffatom gebundenen monocyclischen 5- oder 6-gliedrigen gegebenenfalls partiell gesättigten Heteroarylrest mit 1-4 Ringstickstoffatom und gegebenenfalls einem zusätzlichen Ringheteroatom ausgewählt aus der Gruppe Sauerstoff und Schwefel, oder einen durch einen über ein Ringstickstoffatom gebundenen monocyclischen, 5- oder 6-gliedrigen gegebenenfalls partiell gesättigten Heteroarylrest mit 1-4 Ringstickoffatom substituierten Niederalkylrest darstellt, welche Reste $R_5$ unsubstituiert oder durch Hydroxy, Niederalkoxy, Niederalkanoyloxy, Halogen, Mercapto, Niederalkylthio, gegebenenfalls durch Hydroxy, Niederalkoxy, Carboxy, Niederalkoxycarbonyl, Carbamoyl, gegebenenfalls N-niederalkyliertes Amino, Niederalkylenamino, Niederalkanoylamino, gegebenenfalls durch Amino und/oder Carboxy substituiertes Niederalkoxycarbonylamino, Mecapto, Niederalkylthio oder Sulfo substituiertes Niederalkyl; Amino, Niederalkylamino, Diniederalkylamino, Niederalkylenamino, Guanidino, Ureido, Niederalkanoylamino, gegebenenfalls durch Amino und/oder Carboxy substituiertes Niederalkoxy carbonylamino, Carboxyl, Niederalkoxycarbonyl, Carbamoyl, N-mono-oder N,N-diniederalkyliertes Carbamoyl, Cyano, Sulfo, Sulfamoyl, gegebenenfalls durch Niederalkyl, Nitro, Amino, Hydroxy, Niederalkoxy, Carboxy und/oder Halogen substituiertes Phenyl, Nitro, Oxo und/oder Oxido substituiert sind; oder worin $R_5$ zusammen mit $R_3$ gegebenenfalls durch Hydroxy oder Oxo substituiertes Niederalkylen bedeutet, und $R_6$ Wasserstoff oder Niederalkyl bedeutet, die reinen optischen Isomere von Verbindungen der Formel I, welche im Rest $R_1$ und/oder $R_o$ Chiralitätszentren besitzen, Mischungen dieser optischen Isomere und Salze von solchen Verbindungen der Formel I, die eine salzbildende Gruppe aufweisen.

Die Erfindung betrifft hauptsächlich Verbindungen der Formel (I), worin $R_1$ in $\alpha$-Stellung durch Hydroxy, Triniederalkylsilyloxy, Niederalkenyloxyoxalyloxy oder Niederalkenyloxycarbonyloxy substituiertes Niederalkyl bedeutet, $R_2$ Carboxyl, 4-Nitrobenzyloxycarbonyl, Niederalkenyloxycarbonyl, in 2-Stellung durch Triniederalkylsilyl substituiertes Aethoxycarbonyl oder eine unter physiologischen Bedingungen spaltbare veresterte Carboxylgruppe ist und $R_o$für den Acylrest einer genetisch enkodierten Aminosäure oder für den Acylrest von $\beta$-Alanin, Homoserin, 2-, 3- oder 4-Aminobuttersäure, 2,3-Diaminopropionsäure, 2,3-Diaminobuttersäure, 5-Aminopentansäure, 6-Aminohexansäure, 8-Aminooctansäure, 11-Aminoundecansäure, $\alpha$- oder $\beta$-Aminoisobuttersäure, 3-Amino-3-methyl-buttersäure, 4-Amino-4-methyl-pentansäure, 4-Amino-1-methyl-buttersäure, Aminopivalinsäure, 4-Aminopentansäure, 3-Amino-3-phenyl-propionsäure, Citrullin, Ornithin, Piperidin-3-yl-carbonsäure, Piperidin-4-yl-carbonsäure, 3-Hydroxyprolin oder Pyrrolidin-5-on-2-carbonsäure oder für ein N-Niederalkyl-Derivat eines solchen Acylrestes steht, die reinen Diastereomere von Verbindungen der Formel (I), die im Rest $R_1$ und/oder $R_o$ Chiralitätszentren besitzen, Mischungen solcher Diastereomere, und Salze von solchen Verbindungen der Formel (I), die eine salzbildende Gruppe aufweisen.

Die Erfindung betrifft vor allem Verbindungen der Formel (I), worin $R_1$ Hydroxymethyl oder 1-Hydroxyäthyl ist, $R_2$ Carboxyl oder unter physiologischen Bedingungen spaltbares verestertes Carboxyl bedeutet und $R_o$ für Glycyl, Alanyl, Lysyl, $\beta$-Alanyl, 2,3-Diaminopropionyl, 3-Amino-3-methyl-butryl, 4-Aminobutyryl oder für eine N-Methyl-Derivat eines solchen Aminoacyl-Restes steht, die reinen Diastereomeren von Verbindungen der Formel (I), worin $R_1$ 1-Hydroxyäthyl ist und/oder die im Rest $R_o$ ein Chiralitätszentrum besitzen, Mischungen solcher Diastereomere, und Salze von solchen Verbindungen der Formel (I), die eine salzbildende Gruppe aufweisen.

Die Erfindung betrifft in erster Linie Verbindungen der Formel I, worin $R_1$ 1-Hydroxyäthyl ist, $R_2$ Carboxyl oder unter physiologischen Bedingungen spaltbares verestertes Carboxyl bedeutet und $R_o$ für $\beta$-Alanyl, 3-Amino-3-methyl-butyryl oder 4-Aminobutyryl steht, und Salze davon.

Die Erfindung betrifft vor allem die in den Beispielen genannten Verbindungen der Formel I und ihre pharmazeutisch annehmbaren Salze.

Die Verbindungen der vorliegenden Erfindung können nach an sich bekannten Verfahren hergestellt werden.

Die neuen Verbindungen werden z.B. hergestellt, indem man

a) in einer Verbindung der Formel

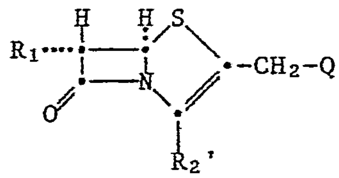

(II),

worin $R_1$ die unter Formel (I) angegebene Bedeutung hat, $R_2'$ eine geschützte Carboxylgruppe ist und Q eine in $R_o$-C(=O)-O- überführbare Gruppe ist, die Gruppe Q in $R_o$-C(=O)-O- überführt, oder

b) eine Ylid-Verbindung der Formel

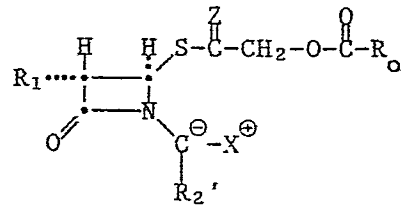

(III),

worin $R_1$ und $R_o$ die unter Formel I angegebenen Bedeutungen haben, $R_2'$ eine geschützte Carboxylgruppe ist, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

c) eine Verbindung der Formel

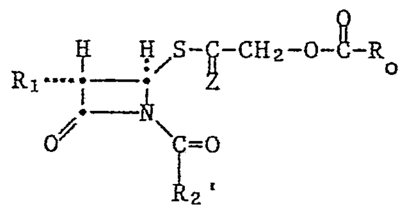

(IV),

worin $R_1$ und $R_o$ die unter Formel I angegebenen Bedeutungen haben, Z Sauerstoff oder Schwefel ist und $R_2'$ eine geschützte Carboxylgruppe ist, mit einer organischen Verbindung des dreiwertigen Phosphors behandelt, und wenn erwünscht oder notwendig, in einer erhältlichen Verbindung der Formel I eine geschützte funktionelle Gruppen in die freie funktionelle Gruppe überführt, und/oder, wenn erwünscht, in einer erhältlichen Verbindung der Formel I eine freie Carobxylgruppe $R_2$ in eine unter physiologischen Bedingungen spaltbare veresterte Carboxylgruppe überführt, und/ oder, wenn erwünscht, in einer erhältlichen Verbindung der Formel I einen Rest $R_o$ in einen anderen Rest $R_o$ überführt, und/oder, wenn erwünscht, ein erhältliches Gemisch von isomeren Verbindungen der Formel I in die einzelnen Isomere auftrennt, und/oder, wenn erwünscht, eine erhältliche Verbindung mit salzbildender Gruppe in ein Salz oder ein erhältliches Salz in die freie Verbindung oder in ein anderes Salz überführt.

In den Ausgangsverbindungen der Formeln II-IV sind funktionelle Gruppen, wie freie Hydroxygruppen, z.B. im Rest $R_1$, und freie Aminogruppen vorzugsweise durch konventionelle Schutzgruppen, z.B. durch eine der oben genannten, geschützt.

a) Substitution des Restes Q

Eine in einen Rest $R_o$-C(=O)-O- überführbare Q ist insbesondere Hydroxy oder eine durch nucleophile Reaktion austauschbare Gruppe. Durch nucleophile Reaktion austauschbare Gruppen Q sind insbesondere veresterte Hydroxygruppen, wie durch eine Halogenwasserstoffsäure, eine Diniederalkyl- oder Diarylphosphorsäure, eine gegebenenfalls durch Oxo oder Halogen substituierte Niederalkancarbonsäure, eine gegebenenfalls durch Halogen substituierte Niederalkan- oder eine gegebenenfalls durch Halogen oder Niederalkyl substituierte Benzolsulfonsäure veresterte Hydroxygruppen. Solche Gruppen Q sind z.B. Halogen, wie Chlor, Brom oder Jod, Diniederalkyl- oder Diarylphosphoryloxy, z.B. Dimethyl-, Diäthyl- oder Diphenylphosphoryloxy, gegebenenfalls durch Halogen oder Oxo substituiertes Niederalkanoyloxy, z.B. Formyloxy, Acetyloxy oder Acetacetyloxy, gegebenenfalls durch Halogen substituiertes Niederalkansulfonyloxy, z.B. Methansulfonyloxy oder Trifluormethansulfonyloxy, oder gegebenenfalls durch Halogen oder Niederalkyl substituiertes Benzolsulfonyloxy, z.B. Benzolsulfonyloxy, 4-Chlor-, 4-Brom- oder 4-Methylbenzolsulfonyloxy.

Die Umsetzung einer Verbindung der Formel (II) worin Q eine durch nucleophile Reaktion austauschbare Gruppe ist, erfolgt z.B. mit einer Carbonsäure der Formel $R_o$-COOH (V) oder zur Herstellung einer Verbindung

der Formel I, worin $R_o$ für die Gruppe -Y-N($R_3$,$R_4$) steht, mit einer Carbonsäure der Formel

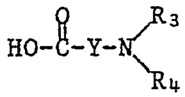

(V'),

wobei funktionelle Gruppen im Rest $R_o$ bzw. in den Resten $R_3$, $R_4$ und Y in geschützter Form vorliegen, zur Bildung eines Salzes derselben in Gegenwart mindestens äquimolarer Mengen eines basischen Kondensationsmittels, beispielsweise eines Alkalimetall-niederalkanolats, z.B. Natrim-methanolat oder Kalium-tert.butanolat, oder eines Alkalimetallhydrids oder -amids, z.B. Natriumhydrid oder Natriumamid, in einem inerten Lösungsmittel, z.B. einem Aether, wie Dioxan oder Tetrahydrofuran, oder einem Amid, z.B. Formamid oder Dimethylformamid, bei Verwendung von Alkalimetall-niederalkanolaten auch im entsprechenden Niederalkanol, oder Lösungsmittelgemisch bei Raumtemperatur oder erniedrigter oder leicht erhöhter Temperatur, z.B. bei etwa -60°C bis etwa +40°C, insbesondere bei etwa -20°C bis etwa +30°C, falls erforderlich in einer Inertgas-, wie Stickstoffatmosphäre.

Man kann auch in einer Verbindung der Formel II, worin Q Hydroxy ist, die Hydroxygruppe Q durch Umsetzung mit einem den Acylrest einer Carbonsäure der Formel V bzw. V′ einführenden Acylierungsmittel acylieren.

Ein den Acylrest einer Carbonsäure der Formel V bzw. V′ einführendes Acylierungsmittel ist entweder die Carbonsäure der Formel V bzw. V′ selbst oder eine reaktionsfähiges funktionelles Derivat davon.

Falls eine freie Säure der Formel V bzw. V′ zur Acylierung eingesetzt wird, wird die Reation üblicherweise in Gegenwart von geeigneten Kondensationsmitteln, wie Carbodiimiden, beispielsweise N,N′-Dicyclohexylcarbodiimid, geeigneten Carbonylverbindungen, beispielsweise Carbonyldiimidazol, oder 1,2-Oxazoliumverbindungen, wie 2-Aethyl-5-phenyl-1,2-oxazolium-3′-sulfonat oder 2-tert.-Butyl-5-methyl-1,2-oxazoliumperchlorat, oder einer geeigneten Acylaminoverbindung, z.B. 2-Aethoxy-1-äthoxycarbonyl-1,2-dihydro-chinolin, gegebenenfalls in Gegenwart einer Base, wie eines Pyridin-Derivats, z.B. 4-Dimethylaminopyridin, durchgeführt.

Ein reaktionsfähiges, d.h. ein Ester-bildendes, funktionelles Derivat einer Carbonsäure der Formel V bzw. V′ ist in erster Linie ein Anhydrid, vorzugsweise ein gemischtes Anhydrid. Ein gemischtes Anhydrid wird beispielsweise durch Kondensation mit einer anderen Säure, z.B. einer anorganischen Säure, z.B. einer Halogenwasserstoffsäure, gebildet und ist beispielsweise das entsprechende Carbonsäurehalogenid, z.B. das Carbonsäurechlorid oder -bromid. Ein gemischtes Anhydrid wird ferner durch Kondensation mit Stickstoffwasserstoffsäure gebildet und ist beispielsweise das Carbonsäureazid. Weitere anorganische Säuren, die sich zur Bildung gemischter Anhydride eignen, sind Phosphor-haltige Säuren, z.B. Phosphorsäure, Diäthylphosphorsäure oder phosphorige Säure, Schwefel-haltige Säuren, z.B. Schwefelsäure, oder Cyanwasserstoffsäure.

Ein reaktionsfähiges funktionelles Derivat einer Carbonsäure der Formel V bzw. V′ ist ebenfalls ein entsprechender aktivierter Ester, der beispielsweise durch Kondensation mit einem vinylogen Alkohol, d.h. mit einem Enol, z.B. einem vinylogen Niederalkenol, gebildet wird, ein Iminomethylesterhalogenid, z.B. Dimethyliminomethylesterchlorid, hergestellt aus der Carbonsäure der Formel V bzw. V′ und z.B. Dimethyl-(1-chloräthyliden)-iminiumchlorid der Formel $[(CH_3)_2N^{\oplus}=C(Cl)CH_3]Cl^{\ominus}$, das man seinerseits z.B. aus N,N-Dimethylacetamid und Phosgen oder Oxalylchlorid erhalten kann, ein Arylester, z.B. ein durch Halogen, z.B. Chlor, und/oder Nitro substituierter Phenylester, z.B. ein Pentachlor-, 4-Nitrophenyl- oder 2,4-Dinitrophenylester, ein N-heteroaromatischer Ester, z.B. N-Benztriazolester, oder ein N-Diacyliminoester, z.B. ein N-Succinimino- oder N-Phthaliminoester.

Die Acylierung mit einem reaktionsfähigen funktionellen Derivat der Carbonsäure der Formel V bzw. V′, z.B. mit einem entsprechenden Anhydrid, insbesondere einem Säurehalogenid, wird bevorzugt in Anwesenheit eines geeigneten säurebindenden Mittels, beispielsweise einer geeigneten organischen Base, durchgeführt. Eine geeignete organische Base ist beispielsweise ein Amin, z.B. ein tertiäres Amin, wie Triniederalkylamin, z.B. Trimethylamin, Triäthylamin oder Aethyl-diisopropylamin, oder ein N,N-Diniederalkylanilin, z.B. N,N-Dimethylanilin, oder ein cyclisches tertiäres Amin, z.B. ein N-niederalkyliertes Morpholin, z.B. N-Methylmorpholin, oder ist beispielsweise eine Base vom Pyridin-Typ, z.B. Pyridin. Ein geeignetes säurebindendes Mittel ist ferner eine anorganische Base, beispielsweise ein Alkalimetall- oder Erdalkalimetallhydroxid, -carbonat oder -hydrogencarbonat, z.B. Natrium-, Kalium- oder Calciumhydroxid, -carbonat oder -hydrogencarbonat.

Die Acylierung mit einer Carbonsäure der Formel V bzw. V′ oder einem reaktionsfähigen, funktionellen Derivat davon wird vorzugsweise in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, cyclischen Aether, z.B. Tetrahydrofuran, einem Ester, z.B. Essigsäureäthylester, oder einem Nitril, z.B. Acetonitril, oder in Mischungen davon, wenn notwendig oder erwünscht, bei erniedrigter oder erhöhter Temperatur, z.B. in einem Temperaturbereich von etwa -40°C bis etwa +100°C, bevorzugt von etwa -20°C bis etwa +50°C, und, falls erforderlich, in einer Inertgas-, z.B. Stickstoffatmosphäre.

In den Ausgangsverbindungen der Formel V bzw. V′ sind vorhandene funktionelle Gruppen in den Resten $R_1$, $R_3$, $R_4$ und Y, z.B. Carboxyl-, Amino- oder Hydroxylgruppen, durch eine der weiter vorn genannten

Schutzgruppen, z.B. Carboxyl-, Amino- oder Hydroxylschutzgruppen, geschützt.

b) Cyclisierung der Verbindung der Formel III

Die Gruppe $X^{\oplus}$ im Ausgangsmaterial der Formel III ist eine der bei Wittig-Kondensationsreaktionen gebräuchlichen Phosphonio- oder Phosphonogruppen, insbesondere eine Triaryl-, z.B. Triphenyl-, oder Triniederalkyl-, z.B. Tri-n-butylphosphoniogruppe, oder eine durch Niederalkyl, z.B. Aethyl, zweifach veresterte Phosphonogruppe, wobei das Symbol $X^{\oplus}$ für den Fall der Phosphonogruppe zusätzlich das Kation einer starken Base, insbesondere ein geeignetes Metall-, wie Alkalimetallion, z.B. das Lithium-, Natrium- oder Kaliumion, umfasst. Bevorzugt als Gruppe $X^{\oplus}$ sind einerseits Triphenylphosphonio und andererseits Diäthylphosphono zusammen mit einem Alkalimetall-, z.B. dem Natriumion.

In Phosphonio-Verbindungen der Formel III wird die negative Ladung durch die positiv geladene Phosphoniogruppe neutralisiert. In Phosphono-Verbindungen der Formel III wird die negative Ladung durch das Kation einer starken Base neutralisiert, das je nach Herstellungsweise des Phosphono-Ausgangsmaterials z.B. ein Alkalimetall-, z.B. Natrium-, Lithium- oder Kaliumion, sein kann. Die Phosphono-Ausgangsstoffe werden daher als Salze in die Reaktion eingesetzt.

Der Ringschluss kann spontan, d.h. bei der Herstellung der Ausgangsstoffe, oder durch Erwärmen, z.B. in einem Temperaturbereich von etwa 30°C bis 160°C, vorzugsweise von etwa 80°C bis etwa 110°C, erfolgen. Die Reaktion wird vorzugsweise in einem geeigneten inerten Lösungsmittel, wie in einem aliphatischen, cycloaliphatischen oder aromatischen Kohlenwasserstoff, z.B. Cyclohexan, Benzol oder Toluol, einem halogenierten Kohlenwasserstoff, z.B. Methylenchlorid, oder einem Aether, z.B. Diäthyläther, Dioxan oder Tetrahydrofuran, oder in einem Gemisch davon, und, falls notwendig, in einer Inertgas-, z.B. Stickstoffatomosphäre, durchgeführt.

c. Cyclisierung der Verbindung der Formel IV

Eine organische Verbindung des dreiwertigen Phosphors leitet sich z.B. von phosphoriger Säure ab und ist insbesondere ein Ester derselben mit einem Niederalkanol, z.B. Methanol oder Aethanol, und/oder einer gegebenenfalls substituierten aromatischen Hydroxyverbindung, z.B. Phenol oder Brenzcatechin, oder ein Amidester derselben der Formel $P(OR_a)_2N(R_b)_2$, worin $R_a$ und $R_b$ unabhängig voneinander Niederalkyl, z.B. Methyl, oder Aryl, z.B. Phenyl, bedeuten. Bevorzugte Verbindungen des dreiwertigen Phosphors sind Triniederalkylphosphite, z.B. Trimethylphosphit oder Triäthylphosphit.

Die Reaktion wird vorzugweise in einem inerten Lösungsmittel, wie einem aromatischen Kohlenwasserstoff, z.B. Benzol oder Toluol, einem Aether, z.B. Dioxan oder Tetrahydrofuran, oder einem halogenierten Kohlenwasserstoff, z.B. Methylenchlorid oder Chloroform, bei einer Temperatur von etwa 20° bis 140°C, bevorzugt bei etwa 40° bis etwa 110°C, durchgeführt, wobei man 1 Moläquivalent einer Verbindung der Formel IV mit 2 Moläquivalenten der Phosphorverbindung umsezt. Vorzugsweise legt man die Verbindung der Formel IV in einem inerten Lösungsmittel vor und tropft die Phosphorverbindung, vorzugsweise in dem gleichen inerten Lösungsmittel gelöst, über einen längeren Zeitraum, z.B. während eines Zeitraums von 2 bis 4 Stunden, hinzu.

In einer bevorzugten Ausführungsform des Verfahrens stellt man das Ausgangsmaterial der Formel IV wie weiter unten angegeben her und setzt es ohne Isolierung aus dem Reaktionsgemisch mit der organischen Verbindung des dreiwertigen Phosphors um, wobei die Endprodukte der Formel I entstehen.

Bevorzugt werden solche Ausgangsmaterialien der Formeln II-V verwendet, die zu den eingangs als besonders bevorzugt genannten Verbindungen der Formel I führen.

In einer erhältlichen Verbindung der Formel I, worin eine oder mehrere funktionelle Gruppen geschützt sind, können diese, z.B. geschützte Carboxyl-, Hydroxy- und/oder Aminogruppen, in an sich bekannter Weise mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

In einer verfahrensgemäss erhältlichen Verbindung der Formel I, worin $R_2$ eine geschützte Carboxylgruppe bedeutet und/oder worin der Rest $R_o$ geschütztes Carboxyl als Substituenten enthält, kann die geschützte Carboxylgruppe in an sich bekannter Weise freigesetzt werden. So kann man in 2-Stellung durch eine trisubstituierte Silylgruppe substituiertes Aethoxycarbonyl z.B. durch Behandeln mit einer Carbonsäure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, gespalten werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zinn, üblicherweise in Gegenwart eines Wasserstoff-abgebenden Mittels, das zusammen mit dem Metal nascierdenden Wasserstoff zu erzeugen vermag, wie einer geeigneten Carbonsäure, z.B. einer gegebenenfalls, z.B. durch Hydroxy, substituierten Niederalkancarbonsäure, z.B. Essigsäure, oder eines Alkohols oder Thiols, wobei man vorzugsweise Wasser zugibt, in freies Carboxyl überführen. Die Abspaltung einer Allylschutzgruppe kann z.B. durch Umsetzung mit einer Verbindung des Palladiums, z.B. Tetrakis-(triphenylphsophin)-palladium, gegebenenfalls in Gegenwart von Triphenylphosphin und unter Zusatz eines Allylgruppenakzeptors, wie einer Carbonsäure, z.B. 2-Aethylhexansäure, oder eines Salzes davon oder Dimedon oder Tributylzinnhydrid, erfolgen. Durch Behandeln mit einem reduzierenden Metall oder Metallsalz, wie oben beschrieben, kann man

auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Benzoylmethoxycarbonyl in freies Carboxyl umwandeln, während Benzoylmethoxycarbonyl ebenfalls durch Behandeln mit einem nucleophilen, vorzugsweise salzbildenden Reagenz, wie Natriumthiophenolat oder Natriumjodid, gespalten werden kann. Substituiertes 2-Silyläthoxycarbonyl kann auch durch Behandeln mit einem das Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetrabutylammoniumfluorid, in freies Carboxyl überführt werden. Eine in 2-Stellung durch Niederalkylsulfonyl oder Cyano substituierte Niederalkoxycarbonylgruppe kann z.B. durch Behandeln mit einem basischen Mittel, wie einem Alkalimetall- oder Erdalkalimetallhydroxid oder -carbonat, z.B. Natrium- oder Kaliumhydroxid oder Natrium- oder Kaliumcarbonat, in freies Carboxyl übergeführt werden.

Andererseits können auch Verbindungen der Formel I, worin $R_2$ Carboxyl, bedeutet, in Verbindungen der Formel I überführt werden, worin $R_2$ eine unter physiologischen Bedingungen spaltbare veresterte Carboxylgruppe darstellt. So kann man die freie Carboxylgruppe z.B. durch Umsetzen mit einem zur Veresterung geeigneten Alkohol in Gegenwart eines Veresterungsmittels, wie eines Carboxiimids, z.B. Dicyclohexylcarbodiimid, oder Carboxyldiimidazol, verestern. Ester können auch durch Umsetzung eines gegebenenfalls in situ hergestellten Salzes der Säure mit einm reaktionsfähigen Ester eines Alkohols und einer starken anorganischen Säure, wie Schwefelsäure, hergestellt werden.

In verfahrensgemäss erhältlichen Verbindungen der Formel I, worin der Rest $R_1$ und gegebenenfalls der Rest $R_o$ geschütztes Hydroxyl als Substituenten enthält, kann die geschützte Hydroxygruppe in an sich bekannter Weise in die freie Hydroxygruppe überführt werden. Beispielsweise wird eine durch eine geeignete Acylgruppe oder eine organische Silylgruppe geschützte Hydroxygruppe wie eine entsprechend geschützte Aminogruppe freigesetzt (s.u.); eine Triniederalkylsilylgruppe kann z.B. auch mit Tetrabutylammoniumfluorid und Essigsäure abgespalten werden (unter diesen Bedingungen werden durch trisubstituiertes Silyläthoxy geschützte Carboxygruppen nicht gespalten).

In einer erfindungsgemäss erhältlichen Verbindung der Formel I mit einer geschützen Aminogruppe kann diese in an sich bekannter Weise, z.B. je nach Art der Schutzgruppe, vorzugsweise mittels Solvolyse oder Reduktion, in die freie Aminogruppe übergeführt werden. Beispielsweise kann 2-Halogenniederalkoxycarbonylamino (gegebenenfalls nach Umwandlung einer 2-Bromniederalkoxycarbonylaminogruppe in eine 2-Jodniederalkoxycarbonylaminogruppe) oder 4-Nitrobenzyloxycarbonylamino durch Behandeln mit einem geeigneten chemischen Reduktionsmittel, wie Zink in Gegenwart einer geeigneten Carbonsäure, wie wässriger Essigsäure, oder katalytisch mit Wasserstoff in Gegenwart eines Palladiumkatalysators gespalten werden. 4-Nitrobenzyloxycarbonylamino kann auch durch Behandeln mit einem Alkalimetall-, z.B. Natriumdithionit gespalten werden. Gegebenenfalls substituiertes Benzyloxycarbonylamino kann z.B. mittels Hydrogenolyse, d.h. durch Behandeln mit Wasserstoff in Gegenwart eines geeigneten Hydrierkatalysators, wie eine Palladiumkatalysators, und Allyloxycarbonylamino durch Umsetzen mit einer Verbindung des Palladiums, z.B. Tetrakis(triphenylphosphin)palladium gegebenenfalls in Gegenwart von Triphenylphosphin und in Gegenwart eines Allylgruppenakzeptors, wie einer Carbonsäure, z.B. 2-Aethylhexansäure, oder eines Salzes davon oder Dimedon oder Tributylzinnhydrid, gespalten werden. Eine durch 2-Halogenniederalkanoyl, z.B. 2-Chloracetyl, geschützte Aminogruppe kann durch Behandeln mit Thioharnstoff in Gegenwart einer Base oder mit einem Salz, wie einem Alkalimetallsalz, des Thioharnstoffs und anschliessender Solvolyse, wie Alkoholyse oder Hydrolyse, des entstandenen Kondensationsproduktes freigesetzt werden. Eine durch 2-substituiertes Silyläthoxycarbonyl geschützte Aminogruppe kann durch Behandeln mit einem Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetraäthylammoniumfluorid, in die freie Aminogruppe übergeführt werden. Eine in Form einer Azidogruppe geschützte Aminogruppe wird z.B. durch Reduktion in freies Amino überführt, beispielsweise durch katalytische Hydrierung mit Wasserstoff in Gegenwart eines Hydrierkatalysators, wie Platinoxid oder Palladium oder durch Behandeln mit Zink in Gegenwart einer Säure, wie Essigsäure.

In Verbindungen der Formel I, worin $R_o$ für die Gruppe $-Y-N(R_3,R_4)$ steht, kann man weiterhin einen Rest $R_3$ und/oder $R_4$ und/oder Y in einen anderen Rest $R_3$ und/oder $R_4$ und/oder Y überführen.

So kann beispielsweise in Verbindungen der Formel I, worin der Rest Y durch eine Carboxylgruppe substituierte ist, diese Carboxylgruppe nach an sich bekannten Verfahren in eine funktionell abgewandelte Carboxylgruppe, wie z.B. in eine veresterte Carboxylgruppe oder in gegebenenfalls substituiertes Carbamoyl, überführt werden. Beispielsweise erhält man durch Umsetzen einer Verbindung der Formel I, worin Y eine Gruppe der Formel $-CR_5R_6-$ ist und $R_5$ ein durch Carboxy substituierter organischer Rest ist, mit einem Niederalkanol eine Verbindung der Formel I, worin Y eine Gruppe der Formel $-CR_5R_6-$ ist und $R_5$ ein durch Niederalkoxycarbonyl substituierter organischer Rest ist, wobei man vorzugsweise in Gegenwart eines geeigneten Kondensationsmittels, z.B. eines Carbodiimids, arbeitet oder das entstehende Wasser z.B. durch azeotrope Destillation entfernt. Andererseits kann man Carboxylgruppen im Rest Y auch in reaktionsfähige funktionelle Derivate, wie gemischte Anhydride, z.B. Säurehalogenide, oder aktivierte Ester, überführen und diese durch Umsetzen mit einem Niederalkanol, Ammoniak oder einem primären oder sekundären Amin, z.B. einem Niederalkyl- oder Diniederalkylamin, in entsprechend veresterte oder amidierte Carboxylgruppen umwandeln, wobei man bei Verwendung von gemischten Anhydriden vorzugsweise in Gegenwart eines säurebindenden Mittels, wie eines aromatischen oder tertiären Amins oder eines Alkalimetall-oder

Erdalkalimetallcarbonats, arbeitet.

Weist eine (Hetero)aryl-Gruppe im Rest $R_o$ eine Hydroxygruppe auf, so lässt sich diese auf üblichem Wege veräthern. Die Umsetzung zu den entsprechenden Niederalkyl-(hetero)aryl-äthern erfolgt beispiels weise in Gegenwart von Basen, wie Alkalimetallhydroxiden oder -carbonaten, z.B. Natriumhydroxid oder Kaliumcarbonat, mit Hilfe von Diniederalkylsulfaten oder Niederalkylhalogeniden. Weiterhin lässt sich Hydroxy in verestertes Hydroxy, z.B. Niederalkanoyloxy, umwandeln, beispielsweise durch Umsetzung mit dem reaktionsfähigen Derivat einer entsprechenden Niederalkancarbonsäure, z.B. Essigsäure, wie einem Anhydrid davon, z.B. dem symmetrischen Anhydrid davon oder einem gemischten Anhydrid mit einer Halogenwasserstoffsäure, erforderlichenfalls in Gegenwart eines basischen Kondensationsmittels, wie eines Alkalimetallhydroxids oder -carbonats, oder einer Stickstoffbase, z.B. Pyridin. Die Umwandlung von Niederalkanoyloxy zu Hydroxy erfolgt beispielsweise durch Alkoholyse oder, vorzugsweise, Hydrolyse, z.B. durch basenkatalysierte Hydrolyse, z.B. in Gegenwart von Natriumhydroxid.

In Verbindungen der Formel I mit einer freien Aminogruppe kann die Aminogruppe in eine substituierte Aminogruppe, z.B. eine Niederalkylamino-, Diniederalkylamino-, Niederalkylenamino-oder Niederalkanoylaminogruppe, überführt werden. Die Ueberführung in eine Niederalkylamino- oder Diniederalkylaminogruppe erfolgt beispielsweise durch Reaktion mit einem reaktionsfähigen veresterten Niederalkanol, beispielsweise einem Niederalkylhalogenid oder -sulfonat, in Gegenwart eines basischen Kondensationsmittels, wie eines Hydroxids oder Carbonats eines Alkali-oder Erdalkalimetalls oder einer heteroaromatischen Stickstoffbase, z.B. Pyridin. In analoger Weise kann Amino durch Behandeln mit einem Niederalkylendihalogenid oder -disulfonat in Niederalkylenamino und durch Behandeln mit dem reaktionsfähigen funtionellen Derivat einer Niederalkancarbonsäure, z.B. dem entsprechenden Carbonsäurehalogenid, in Niederalkanoylamino umgewandelt werden. Verbindungen der Formel I mit einem substituierbaren Ringstickstoffatom im Rest $R_o$ können auf die gleiche Weise in Verbindungen der Formel I überführt werden, welche im Rest $R_o$ ein durch einen gegebenenfalls substituierten Niederalkylrest substituiertes Ringstickstoffatom enthalten. Weiterhin können Verbindungen der Formel I, worin $R_4$ Wasserstoff ist, durch Umsetzen mit einem den Acylrest einer Carbonsäure, eines Halbesters der Kohlensäure, einer gegebenenfalls substituierten Carbaminsäure, Sulfonsäure oder Amidosulfonsäure, z.B. einem Säurehalogenid einer solchen Säure, z.B. dem Chlorid oder Bromid davon, einführenden Acylierungsmittel vorzugsweise in Gegenwart eines säurebindenden Mittels, wie eines der oben genannten, in Verbindungen der Formel I überführt werden, worin $R_4$ Acyl ist.

Salze von Verbindungen der Formel I mit salzbildenden Gruppen können in an sich bekannter Weise hergestellt werden. So kann man Salze von Verbindungen der Formel I mit einer freien Carboxyl- oder Sulfogruppe z.B. durch Behandeln mit Metallverbindungen, wie Alkalimetallsalzen von geeigneten organischen Carbonsäure, z.B. dem Natriumsalz der $\alpha$-Aethylcapronsäure, oder mit anorganischen Alkali-oder Erdalkalimetallsalzen, z.B. Natriumhydrogencarbonat, oder mit Ammoniak oder mit einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer geeigneten Säure oder einem geeigneten Anionenaustauscherreagenz. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen überführt werden, Metall- und Ammoniumsalze z.B. durch Behandeln mit geeigneten Säuren und Säureadditionssalze z.B. durch Behandeln mit einem geeigneten basischen Mittel.

Erhaltene Gemische von isomeren Verbindungen können nach an sich bekannten Methoden in die einzelnen Isomere aufgetrennt werden. Beispielsweise kann man ein erhaltenes Racemat mit einem optisch aktiven Hilfsstoff reagieren lassen, das dabei entstandene Gemisch zweier diastereomerer Verbindungen mit Hilfe von geeigneten physi kalisch-chemischen Methoden (z.B. fraktioniertes Kristallisieren, Adsorptionschromatographie) trennen und die einzelnen diastereomeren Verbindungen dann in die optisch aktiven Verbindungen spalten.

Bei allen nachträglichen Umwandlungen erhaltener Verbindungen der Formel I werden solche Reaktionen bevorzugt, die unter gemässigt alkalischen oder insbesondere neutralen Bedingungen erfolgen.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird. Ferner können Ausgangsstoffe in Form von Derivaten verwendet oder in situ, gegebenenfalls unter den Reaktionsbedingungen, hergestellt werden.

Die Ausgangsverbindungen der Formeln III und IV können, wie in dem folgenden Reaktionsschema I angegeben, hergestellt werden:

Reaktionsschema I

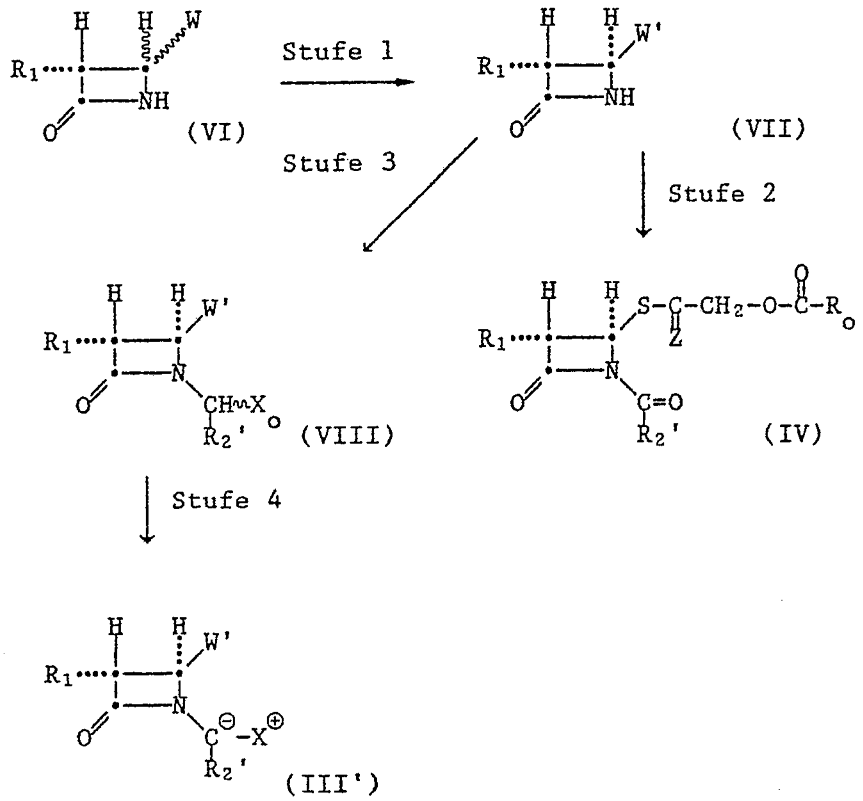


(VI) (VII) (VIII) (IV) (III')

In den Verbindungen der Formeln III', VII und VIII steht W' für $R_o$ oder für Triphenylmethylthio oder Niederalkanoylthio.

Stufe 1

Geeignete Ausgangsverbindungen der Formel VI, worin W ein durch nucleophile Reaktion leicht austauschbarer Rest, z.B. Niederalkanoyloxy, wie Acetyloxy, oder Sulfonyloxy $R\text{-}SO_2\text{-}$, worin R z.B. gegebenenfalls durch Hydroxy substituierten Niederalkyl, wie Methyl, tert. Butyl oder 2-Hydroxyäthyl, ist, sind beispielsweise aus der veröffentlichten Europäischen Patentanmeldung Nr. 82113, der Deutschen Offenlegungsschrift Nr. 3 224 055 und der Deutschen Offenlegungsschrift Nr. 3 013 997 bekannt oder können in dazu analoger Weise hergestellt werden.

Eine den Rest $-S-C(=Z)-CH_2-O-C(=O)-R_o$ einführende Verbindung ist beispielsweise eine Säure der Formel $R_o-C(=O)-O-CH_2C(=Z)-SH$ oder insbesondere ein Salz, z.B. Alkalimetallsalz, wie das Natrium-oder Kaliumsalz, davon. Die Substitution kann in einem organischen Lösungsmittel, wie in einem Niederalkanol, einem Niederalkancarbonsäureamid, einem cyclischen Aether, oder in einem ähnlichen inerten Lösungsmittel bei Raumtemperatur oder bei etwas erhöhter oder erniedrigter Temperatur, z.B. bei etwa 0° bis etwa 40°C, durchgeführt werden. Die Einführung eines Triphenylmethylthio-oder Niederalkanoylthio-Restes W' erfolgt in analoger Weise durch Umsetzen mit einem Alkalimetallsalz, z.B. dem Natriumsalz, einer Niederalkanthiocarbonsäure, z.B. Thioessigsäure, oder des Triphenylmethylmercaptans.

Stufe 2

Eine Ausgangsverbindung der Formel (IV) wird erhalten, indem man ein Azetidinon der Formel (VII), in der W' für den Rest $-S-C(=Z)-CH_2-O-C(=O)-R_o$ steht, mit einer Säure der Formel $R_2'\text{-}COOH$ oder insbesondere einem reaktionsfähigen Derivat, wie einem Ester oder Säurehalogenid, z.B. dem Säurechlorid, davon bei einer Temperatur von -50°C bis 80°C, bevorzugt bei -20°C bis 0°C, in einem inerten Lösungsmittel, wie einem der bei der Umsetzung von Verbindungen der Formel (IV) zu Verbindungen der Formel I genannte, behandelt. Bei Verwendung eines Säurehalogenids arbeitet man vorzugsweise in Gegenwart eines säurebindenden Mittels, wie eines tertiären aliphatischen Amins, eines aromatischen Amins, oder insbesondere eines Alkalimetall- oder Erdalkalimetallcarbonats oder -hydrogencarbonats.

Verbindungen der Formel (VIII), worin W' für Triphenylmethylthio oder Niederalkanoylthio steht, können in die Ausgangsverbindungen der Formel VII, worin W' für den Rest $-S-C(=Z)-CH_2-O-C(=O)-R_o$ steht, überführt werden, indem man sie in Gegenwart einer Base, z.B. Pyridin oder Tri-n-butylamin, in einem

geeigneten Lösungsmittel, z.B. Diäthyläther oder Methanol, mit einem Salz der Formel MA, worin M für ein Uebergangsmetallkation, insbesondere für das Silberkation, steht, und A ein gebräuchliches Anion darstellt, welches die Löslichkeit des Salzes MA in dem gewählten Lösungsmittel begünstigt, z.B. das Nitrat-, Acetat- oder Fluorid-Anion, umsetzt, und das entstandene Salz der Formel

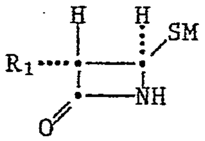

(VII'),

mit einem den Rest $R_o$-C(=O)-O-$CH_2$C(=Z)- einführenden Acylierungsmittel, z.B. mit der Säure $R_o$-C(=O)-O-$CH_2$-C(=Z)-OH oder einem reaktionsfähigen funktionellen Derivat, wie einem Säurehalogenid, z.B. dem Chlorid oder Bromid, Azid oder Anhydrid davon, behandelt. Die Acylierung erfolgt,wenn ein reaktionsfähiges funktionelles Derivat der Säure der Formel $R_o$-C(=O)-O-$CH_2$-C(=Z)-OH, z.B. das Säurechlorid, eingesetzt wird, in einem inerten Lösungsmittel, wie einem chlorierten Kohlenwasserstoff, oder einem Aether, bei Raumtemperatur oder unter Erhitzen oder Kühlen, z.B. in einem Temperaturbereich von ca. -50° bis ca. +60° C, insbesondere bei ca. -30° bis ca. +20° C.

Stufe 3

Verbindungen der Formel VIII, worin $X_o$ für eine reaktionsfähige veresterte Hydroxygruppe, insbesondere für Halogen, z.B. Chlor oder Brom, steht, werden hergestellt, indem man eine Verbindung der Formel VII mit einer Glyoxylsäure-Verbindung der Formel $R_2'$-CHO oder einem geeigneten Derivat davon, wie einem Hydrat, Hemihydrat oder Halbacetal, z.B. einem Halbacetal mit einem Niederalkanol, z.B. Methanol oder Aethanol, umsetzt, und in einer erhaltenen Verbindung der Formel VIII, worin $X_o$ für Hydroxy steht, die Hydroxygruppe in eine reaktionsfähige veresterte Hydroxygruppe umwandelt. Die Verbindungen der Formel VIII werden üblicherweise als Gemisch der beiden Isomere [bezüglich der Gruppierung -CH($R'_2$) $X_o$] erhalten.

Die Anlagerung der Glyoxylsäureester-Verbindung an das Stickstoffatom des Lactamrings in der Verbindung der Formel VII findet bei Raumtemperatur oder, falls notwendig, unter Erwärmen, statt. Bei Verwendung des Hydrats der Glyoxylsäure-Verbindung wird Wasser gebildet, das, wenn notwendig, durch Destillation, z.B. azeotrop, oder durch Verwendung eines geeigneten Dehydratationsmittels enfernt wird. Vorzugsweise arbeitet man in Gegenwart eines geeigneten inerten Lösungsmittels oder Lösungsmittelgemisches.

Die Ueberführung einer Hydroxygruppe $X_o$ in eine reaktionsfähige veresterte Hydroxygruppe $X_o$ in einer Verbindung der Formel VIII wird durch Behandeln mit einem geeigneten Veresterungsmittel durchgeführt, z.B. mit einem Thionylhalogenid, z.B. -chlorid, vorzugsweise in Gegenwart eines basischen, in erster Linie organischen basischen Mittels, wie eines aliphatischen tertiären Amins, oder einer heterocyclischen Base von Pyridintyp. Vorzugsweise arbeitet man in Gegenwart eines geeigneten Lösungsmittels, z.B. Dioxan oder Tetrahydrofuran, oder eines Lösungsmittelgemisches, wenn notwendig unter Kühlen, z.B. bie etwa -30° bis etwa 30° C.

Stufe 4

Das Ausgangsmaterial der Formel III wird erhalten, indem man eine Verbindung der Formel VIII mit einer geeigneten Phosphin-Verbindung, wie einem Triniederalkylphosphin, z.B. Tri-n-butyl-phospin, oder einem Triaryl-phosphin, z.B. Triphenylphosphin, oder mit einer geeigneten Phosphit-Verbindung, wie einem Triniederalkylphosphit, z.B. Triäthylphosphit, oder einem Alkalimetalldiniederalkylphosphit, z.B. -diäthylphosphit, behandelt, und eine gegebenenfalls erhältichen Verbindung der Formel III' worin W' für Triphenylmethylthio oder Niederalkanoylthio steht, in eine Verbindung der Formel III' (=III) überführt, worin W' für den Rest -S-C(=Z)-$CH_2$-O-C(=O)-$R_o$steht.

Die Umsetzung mit der Phosphin- bzw. Phosphit-Verbindung wird vorzugsweise in einem geeigneten inerten Lösungsmittel, wie einem Kohlenwasserstoff, oder einem Aether oder in einem Lösungsmittelgemisch vorgenommen. Je nach Reaktionsfähigkeit arbeitet man unter Kühlen oder bei erhöter Temperatur, etwa zwischen -10° C und +100° C, bevorzugt bei etwa 20° bis 80° C. Ueblicherweise arbeitet man in Gegenwart eines basischen Mittels, wie einer organischen Base, z.B. eines Amins, oder "Polystyol-Hünigbase", oder einer anorganischen Base, z.B. eines Alkalimetallcarbonats, wobei die primär entstandene Phosphoniumverbindung der Formel

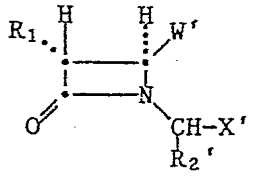

(IIIa),

worin X' eine Phosphonogruppe oder eine Phosphoniogruppe zusammen mit einem Anion, je nach Bedeutung

des Restes $X_o$ (vgl. Formel VIII) z.B. Chlorid, bedeutet, in das Ylid-Ausgangsmaterial der Formel III umgewandelt wird.

Die Einführung des Restes $-S-C(=Z)-CH_2-O-C(=O)-R_o$ in Verbindungen der Formel III', worin W' für Niederalkanoylthio oder Triphenylmethylthio steht, kann in analoger Weise erfolgen, wie unter Stufe 2 beschrieben.

Die Ausgangsverbindungen der Formel II können z.B. hergestellt werden, indem man ein Phosphoran der Formel

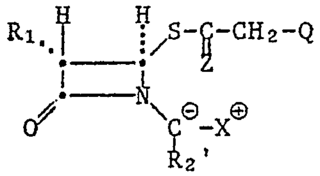

(IX),

welches in analoger Weise, wie im Reaktionsschema I dargestellt, synthetisiert werden kann, ringschliesst, z.B. wie unter Verfahren b) beschrieben.

Man kann das in Reaktionsschema I beschriebene Verfahren zur Herstellung der Verbindungen der Formeln (III), (IV), (VII) und (VIII), sowie das angegebene Verfahren zur Herstellung der Verbindungen der Formel II und der Endprodukte der Formel (I) auch mit optisch inaktiven Verbindungen durchführen und auf einer beliebigen Verfahrensstufe aus einem erhaltenen Diastereomerengemisch oder Racemat in bekannter Weise die optisch aktiven Verbindungen gemäss vorliegender Erfindung isolieren.

Die Erfindung betrifft ebenfalls die neuen Ausgangsverbindungen sowie verfahrensgemäss erhältliche neue Zwischenprodukte, wie diejenigen der Formeln II-IV, ferner VII und VIII [W' steht für den Rest $-S-C(=Z)-CH_2-O-C(=O)-R_o$] sowie die angegebenen Verfahren zu ihrer Herstellung.

Vorzugsweise werden solche Ausgangsverbindungen verwendet und die Reaktionsbedingungen so gewählt, dass man zu den vorstehend als besonders bevorzugt aufgeführten Verbindungen der Formel I gelangt.

Die pharmakologisch verwendbaren Verbindungen der vorliegenden Erfindung können z.B. zur Herstellung von pharmazeutischen Präparaten verwendet werden, welche eine therapeutisch wirksame Menge der Aktivsubstanz zusammen oder im Gemisch mit anorganischen oder organischen, festen oder flüssigen, pharmazeutisch annehmbaren Trägerstoffen enthalten, die sich zur oralen oder zur parenteralen, d.h. z.B. intramuskulären, intravenösen, subcutanen oder intraperitonealen, Verabreichung eignen.

Zur oralen Verabreichung verwendet man Tabletten oder Gelatinekapseln, welche den Wirkstoff zusammen mit Verdünnungsmitteln, z.B. Lactose, Dextrose, Sucrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen. Tabletten enthalten ebenfalls Bindemittel, z.B. Magnesiumaluminiumsilikat, Stärken, wie Mais-, Weizen-, Reis- oder Pfeilwurzstärke, Gelatine, Tragacant, Methylcellulose, Natriumcarboxymethylcellulose und/oder Polyvinylpyrrolidon, und, wenn erwünscht, Sprengmittel, z.B. Stärken, Agar, Alginsäure oder Salze davon, wie Natriumalginat, und/oder Brausemischungen oder Adsorptionsmittel, Farbstoffe, Geschmackstoffe oder Süssmittel.

Zur parenteralen Verabreichung eignen sich in erster Linie Infusionslösungen, vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit enthalten, vor Gebrauch hergestellt werden können. Solche Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten.

Die vorliegenden pharmazeutischen Präparate, die, wenn erwünscht, weitere pharmakologisch wertvolle Stoffe enthalten können, werden in an sich bekannter Weise, z.B. mittels konventioneller Mischungs-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffs.

Je nach Art der Infektion und Zustand des infizierten Organismus verwendet man tägliche Dosen (oral oder parenteral) von etwa 100 mg bis etwa 1 g zur Behandlung von Warmblütern (Menschen und Tiere) von etwa 70 kg Gewicht.

Die folgenden Beispiele dienen zur Illustration der Erfindung. Temperaturen werden in Celsiusgraden angegeben.

Beispiel 1:

(5R,6S)-2-(2,2,2-Trichloräthoxycarbonyloxymethyl)-6-[(1R)1-allyloxycarbonyloxyäthyl]-2-penem-3-carbonsäureallylester

Eine Lösung von 0,46 g 2-[(3S,4R)-3-[(1R)-1-Allyloxycarbonyloxyäthyl]-4-(2,2,2-trichloräthoxycarbonyloxyacetylthio)-2-oxoazetidin-1-yl ]-2-triphenylphosphoranylidenessigsäureallylester in 46 ml Toluol wird unter Argonatmosphäre 3 Stunden bei Rückflusstemperatur gerührt. Dann wird das Lösungsmittel abgedampft und das Rohprodukt durch Chromatographie an Silicagel gereinigt (Laufmittel Toluol/Aethylacetat 9:1). $R_f$ (Toluol/Aethylacetat 1:1) = 0,8. IR ($CH_2Cl_2$): 1795; 1765; 1745; 1710 $cm^{-1}$.

Das Ausgangsmaterial kann wie folgt hergestellt werden:

2-[(3S,4R)-3-[(1R)-1-Allyloxycarbonyloxyäthyl]-4-(2,2,2-trichloräthoxycarbonyloxyacetylthio)-2-oxo-azetidin-1-yl]-2-triphenylphosphoranylidenessigsäureallylester

8 g Silbersalz des 2-[(3S,4R)-3-[(1R)-1-Allyloxycarbonyloxyäthyl]-4-mercapto-2-oxo-azetidin-1-yl]-2-triphenylphosphoranylidenessigsäureallylesters werden in 100 ml Methylenchlorid gelöst, bei 0° mit 1,85 ml Pyridin, 80 mg 4-Dimethylaminopyridin und anschliessend tropfenweise mit einer Lösung von 5,4 g 2,2,2-Trichloräthoxycarbonyloxyacetylchlorid in 50 ml Methylchlorid versetzt. Nach 45 Minuten Rühren bei 0° wird der Feststoff über Hyflo abfiltriert und das Filtrat wird mit wässriger Natriumbicarbonat Lösung und dann mit Sole gewaschen. Nach Trocknen über $MgSO_4$ wird das Lösungsmittel abgedampft. Der Rückstand wird durch Chromatographie an Silicagel (Laufmittel Toluol/Aethylacetat 15:85 bis 1:1) gereinigt.
DC (Toluol/Aethylacetat 1:1) $R_f = 0,5$;
IR ($CH_2Cl_2$): 1765; 1750; 1705; 1620 $cm^{-1}$.

Beispiel 2: (5R,6S)-2-Hydroxymethyl-6-[(1R)-1-allyloxycarbonyloxyäthyl]-2-penem-3-carbonsäureallylester.

Eine Lösung von 0,23 g (5R,6S)-2-(2,2,2-Trichloräthoxycarbonyloxymethyl)-6-[(1R)-1-allyloxycarbonyloxyäthyl]-2-penem-3-carbonsäureallylester in 5 ml abs. Tetrahydrofuran und 0,46 ml einer wässrigen 1M Kaliumdihydrogenphosphat-Lösung wird mit 0,41 g Zinkstaub versetzt und während 5 Stunden bei Raumtemperatur gerührt. Die Suspension wird über Hyflo filtriert und das Filtrat anschliessend unter vermindertem Druck eingeengt. Der Rückstand wird in Aethylacetat aufgenommen, mit wässrigem Natriumbicarbonat und Sole gewaschen, getrocknet ($Na_2SO_4$) und eingeengt. Die Reinigung der Titelsubstanz erfolgt durch Chromatographie an Silicagel. (Laufmittel: Toluol/Aethylacetat 95:5 bis 90:10). DC (Toluol/Aethylacetat 1:1) $R_f = 0,38$;
IR ($CH_2Cl_2$): 3600; 1795; 1750; 1705; 1580 $cm^{-1}$.

Beispiel 3:
(5R,6S)-2-(N-Allyloxycarbonylglycyloxymethyl)-6-[(1R)-1-allyloxycarbonyloxyäthyl]-2-penem-3-carbonsäureallylester

Eine Lösung von 179 mg (5R,6S)-2-Hydroxymethyl-6-[(1R)-1-allyloxycarbonyloxyäthyl]-2-penem-3-carbonsäureallylester in 3 ml abs. Methylenchlorid wird bei Raumtemperatur mit 80 mg N-Allyloxycarbonylglycin und 2 mg 4-dimethylaminopyridin versetzt. Nach Kühlung der Lösung auf 0° werden 113 mg Dicyclohexylcarbodiimid zugegeben und die erhaltene Suspension wird 5 Minuten bei 0° und anschliessend 3 Stunden bei Raumtemperatur gerührt. Der Feststoff wird abfiltriert, nachgewaschen, und das Filtrat wird mit wässrigem Natriumbicarbonat und Sole gewaschen. Nach Trocknen über Natriumsulfat wird die organische Phase vollständig eingedampft. Die Titelsubstanz wird durch Chromatographie an Silicagel gereinigt. (Laufmittel Toluol/Aethylacetat 95:5). DC (Toluol/Aethylacetat 1:1) $R_f = 0,4$.
IR ($CH_2Cl_2$): 3440; 1792; 1745; 1725; 1585 $cm^{-1}$.

Beispiel 4: (5R,6S)-2-Glycyloxymethyl-6-[(1R)-1-hydroxyäthyl]-2-penem-3-carbonsäure

Eine Lösung von 0,66 g (5R,6S)-2-(N-Allyloxycarbonylglycyloxymethyl)-6-[(1R)-1-allyloxycarbonyloxyäthyl]-2-penem-3-carbonsäureallylester in 26 ml abs. THF wird mit 81 mg Tetrakis-triphenylphosphinpalladium und 1,4 ml Tributylzinnhydrid versetzt. Nach 2 Stunden Rühren bei Raumtemperatur wird das Gemisch mit 0,33 ml Essigsäure tropfenweise versetzt und weitere 15 Minuten gerührt. Nach Konzentrieren am Rotationsverdampfer wird der Rückstand in Wasser-Aethylacetat aufgenommen und mittels $NaHCO_3$ neutral gestellt. Die wässrige Phase wird noch zweimal mit Aethylacetat nachgewaschen und am Hochvakuum konzentriert. Die Reinigung durch Chromatographie an Opti $UPC_{12}$ (Laufmittel Wasser) ergibt die Titelsubstanz.
DC (Opti $UPC_{12}$; Wasser) $R_f = 0,43$.
IR (DMSO-$d_6$): 3428; 2969; 1776; 1755; 1619 $cm^{-1}$.
UV (Wasser): $\lambda_{max}$: = 308 nm.

Beispiel 5:
(5R,6S)-2-[3-(N-allyloxycarbonylamino)-propionyloxymethyl]-6-[(1R)-1-Allyloxycarbonyloxyäthyl]-2-penem-3-carbonsäure-allylester

Analog Beispiel 3 werden 213 mg 3-(N-Allyloxycarbonylamino)-propionsäure durch Umsetzung mit (5R,6S)-2-Hydroxymethyl-6-[(1R)-1-allyloxycarbonyloxyäthyl]-2-penem-3-carbonsäureallylester in die Titelverbindung überführt.
IR ($CH_2Cl_2$): 3450; 1795; 1745; 1725; 1240 $cm^{-1}$.

Das Ausgangsmaterial kann wie folgt hergestellt werden:

3-(N-Allyloxycarbonylamino)-propionsäure

Eine Lösung von 17,8 g β-Alanin in 100 ml 5N Natronlauge und 50 ml Wasser wird bei 0° innert 10 Minuten mit 23,3 ml Chlorameisensäureallyester versetzt. Anschliessend wird das Reaktionsgemisch während 12 Stunden bei Raumtemperatur gerührt, zweimal mit Aethylacetat gewaschen und mit 2N HCl auf pH 2 gestellt. Nach zweimaligem Extrahieren mit Aethylacetat werden die vereinigten Extrakte mit Sole gewaschen, über Natriumsulfat getrocknet und eingedampft. IR ($CH_2Cl_2$): 3445; 2950; 1720; 1230 $cm^{-1}$.

Beispiel 6: (5R,6S)-2-(3-Aminopropionyloxymethyl)-6-[(1R)-1-hydroxyäthyl]-2-penem-3-carbonsäure

Analog Beispiel 4 werden 0,43 g (5R,6S)-2-[3-(N-Allyloxycarbonylamino)-propionyloxymethyl]-6-[(1R)-1-allyloxycarbonyloxyäthyl]-2-penem-3-carbonsäureallylester in die Titelverbindung überführt. IR ($DMSO-d_6$): 3300; 2969; 1774; 1736; 1617; 1585; 1356 $cm^{-1}$. UV (Wasser): $\lambda_{max}$: = 308 nm.

Beispiel 7:
(5R,6S)-2-[(2S)-$N_\alpha$,$N_\varepsilon$-Bis-(allyloxycarbonyl)-lysyloxymethyl]-6-[(1R)-1-allyloxycarbonyloxyäthyl]-2-penem-3-carbonsäure-allylester

Analog Beispiel 3 werden 387 mg (2S)-$N_\alpha$,$N_\varepsilon$-Bis-(allyloxycarbonyl)-lysin durch Umsetzung mit (5R,6S)-2-Hydroxymethyl-6-[(1R)-1-allyloxycarbonyloxyäthyl]-2-penem-3-carbonsäureallylester in die Titel verbindung überführt.
IR ($CH_2Cl_2$): 3440; 1795; 1750; 1725; 1240 $cm^{-1}$.
Das Ausgangsmaterial kann wie folgt hergestellt werden:

(2S)-$N_\alpha$,$N_\varepsilon$-Bis-(allyloxycarbonyl)-lysin

Abgesehen davon, das 2 Mol Chlorameisensäureallylester und 1 Mol Aminosäure eingesetzt werden, wird die Titelverbindung in gleicher Weise hergestellt wir 3-(N-Allyloxycarbonylamino)-propionsäure (vgl. Beispiel 5).
IR ($CH_2Cl_2$): 3445; 2960; 1725; 1515 $cm^{-1}$.

Beispiel 8: (5R,6S)-2-[(2S)-Lysyloxymethyl]-6-[(1R)-1-hydroxyäthyl]-2-penem-3-carbonsäure

Analog Beispiel 4 werden 0,55 g (5R,6S)-2-[(2S)-$N_\alpha$,$N_\varepsilon$-Bis-(allyloxycarbonyl)-lysyloxymethyl]-6-[(1R)-1-allyloxycarbonyloxyäthyl]-2-penem-3-carbonsäureallylester in die Titelverbindung überführt. IR ($DMSO-d_6$): 3434; 2934; 1773; 1732; 1617; 1360 $cm^{-1}$.
UV (Wasser): $\lambda_{max}$: = 307 nm.

Beispiel 9:
(5R,6S)-2-[(2R,S)-2,3-Bis-(Allyloxycarbonylamino)-propionyloxymethyl]-6-[(1R)-1-allyloxycarbonyloxyäthyl]-2-penem-3-carbonsäureallylester

Analog zu Beispiel 3 werden 335 mg (2R,S)-2,3-Bis-(allyloxycarbonylamino)-propionsäure in die Titelverbindung überführt.
IR ($CH_2Cl_2$): 3430; 1795; 1720; 1585 $cm^{-1}$.
Abgesehen davon, dass 2 Mol Chlorameisensäureallylester und 1 Mol Aminosäure eingesetzt werden, wird das Ausgangsmaterial, (2R,S)-2,3-Bis-(allyloxycarbonylamino)-propionsäure, in analoger Weise wie 3-(N-Allyloxycarbonylamino)-propionsäure (vgl. Beispiel 5) hergestellt.
IR ($CH_2Cl_2$): 3440; 1710; 1505; 1220 $cm^{-1}$.

Beispiel 10:
(5R,6S)-2-[(2R,S)-2,3-Diaminopropionyloxymethyl]-6-[(1R)-1-hydroxyäthyl]-2-penem-3-carbonsäure

Analog Beispiel 4 werden 0,2 g (5R,6S)-2-[(2R,S)-2,3-Bis(allyloxycarbonylamino)-propionyloxymethyl]-6-[(1R)-1-allyloxycarbonyloxyäthyl]-2-penem-3-carbonsäureallylester in die Titelverbindung überführt.
IR ($DMSO-d_6$): 3430; 2972; 1775; 1750; 1620 $cm^{-1}$.
UV (Wasser): $\lambda_{max}$: = 307 nm.

Beispiel 11:
(5R,6S)-2-[(2S)-1-Allyloxycarbonylpropyloxymethyl]-6-[(1R)-1-allyloxycarbonyloxyäthyl]-2-penem-3-carbonsäureallylester

Analog Beispiel 3 werden 1,5 g (2S)-1-Allyloxycarbonylprolin in die Titelverbindung überführt.
IR ($CH_2Cl_2$): 1792; 1715; 1585 $cm^{-1}$.
Das Ausgangsmaterial, (2S)-1-Allyloxycarbonylprolin, wird in analoger Weise wie 3-(N-allyloxycarbonylamino)-propionsäure (vgl. Beispiel 5) hergestellt.
IR ($CH_2Cl_2$): 2960; 1705; 1410 $cm^{-1}$.

Beispiel 12: (5R,6S)-2-[(2S)-Propyloxymethyl]-6-[(1R)-1-hydroxyäthyl]-2-penem-3-carbonsäure

Analog Beispiel 4 werden 0,7 g (5R,6S)-2-[(2S)-1-Allyloxycarbonylprolyloxymethyl]-6-[(1R)-1-allyloxycarbonyloxyäthyl]-2-penem-3-carbonsäureallylester in die Titelverbindung überführt.
UV (Wasser): $\lambda_{max}$: = 305 nm.

Beispiel 13:
(5R,6S)-6-[(1R)-1-Allyloxycarbonyloxyäthyl]-2-[4-(N-allyloxycarbonylamino)-butyryloxymethyl]-2-penem-3-carbonsäureallylester)

Analog Beispiel 3 werden 0,98 g 4-(N-Allyloxycarbonyl-amino)-buttersäure durch Umsetzung mit (5R,6S)-2-Hydroxymethyl-6-[(1R)-1-allyloxycarbonyloxyäthyl]-2-penem-3-carbonsäureallylester in die Titelverbindung überführt.

IR($CH_2Cl_2$): 3450, 1795, 1750, 1720, 1590 $cm^{-1}$.

Beispiel 14: (5R,6S)-2-(4-Aminobutyryloxymethyl)-6-[(1R)-1-hydroxyäthyl]-2-penem-3-carbonsäure

Analog Beispiel 4 werden 3,15 g (5R,6S)-6-[(1R)-1-Allyloxycarbonyloxyäthyl]-2-[4-(N-allyloxycarbonylamino)-butyryloxymethyl]-2-penem-3-carbonsäureallylester in die Titelverbindung überführt.
IR(DMSO-$d_6$): 3349; 1787; 1742; 1619 $cm^{-1}$,
UV (Wasser): $\lambda_{max}$: = 306 nm.

Beispiel 15:
(5R,6S)-6-[(1R)-1-Allyloxycarbonyloxyäthyl]-2-[5-(N-allyloxycarbonylamino)-pentanoyloxymethyl]-2-penem-3-carbonsäureallylester)

Analog Beispiel 3 werden 0,31 g 5-(N-Allyloxycarbonylamino)-pentansäure durch Umsetzung mit (5R,6S)-2-Hydroxymethyl-6-[(1R)-1-allyloxycarbonyloxyäthyl]-2-penem-3-carbonsäureallylester in die Titelverbindung überführt.
IR ($CH_2Cl_2$): 3430; 1792; 1750; 1718; 1590 $cm^{-1}$.

Beispiel 16: (5R,6S)-2-(5-Aminopentanoyloxymethyl)-6-[(1R)-1-hydroxyäthyl]-2-penem-3-carbonsäure

Analog Beispiel 4 werden 0,5 g (5R,6S)-6-[(1R)-1-Allyloxycarbonyloxyäthyl]-2-[5-(N-allyloxycarbonylamino)-pentanoyloxymethyl]-2-penem-3-carbonsäureallylester) in die Titelverbindung überführt.
IR (DMSO-$d_6$): 3439; 1774; 1733; 1618 $cm^{-1}$
UV (Wasser): $\lambda_{max}$: 307 nm.

Beispiel 17: In analoger Weise, wie in den vorangehenden Beispielen beschrieben, können die folgenden Verbindungen hergestellt werden:
(5R,6S)-6-Hydroxymethyl-2-sarcosyloxymethyl-2-penem-3-carbonsäure, UV (Wasser) $\lambda_{max}$:. = 307 nm.
(5R,6S)-6-Hydroxymethyl-2-glycyloxymethyl-2-penem-3-carbonsäure, UV (Wasser) $\lambda_{max}$:. = 307 nm.
(5R,6S)-6-Hydroxymethyl-2-[(2S)-alanyloxymethyl]-2-penem-3-carbonsäure, UV (Wasser) $\lambda_{max}$:. = 308 nm.
(5R,6S)-6-Hydroxymethyl-2-[(2S)-propyloxymethyl]-2-penem-3-carbonsäure, UV (Wasser) $\lambda_{max}$: = 308nm, IR (DMSO-$d_6$): 3305, 1778, 1620 $cm^{-1}$.
(5R,6S)-2-(6-Aminohexanoyloxymethyl)-6-[(1R)-1-hydroxyäthyl]-2-penem-3-carbonsäure, IR (DMSO-$d_6$): 3435; 1780; 1735; 1618 $cm^{-1}$, UV (Wasser) $\lambda_{max}$: 306 nm.
(5R,6S)-2-(8-Aminooctanoyloxymethyl)-6-[(1R)-hydroxyäthyl]-2-penem-3-carbonsäure, IR (DMSO-$d_6$): 3437; 1776; 1740; 1620 $cm^{-1}$, UV (Wasser) $\lambda_{max}$: 307 nm.
(5R,6S)-2-(11-Aminoundecanoyloxymethyl)-6-[(1R)-1-hydroxyäthyl]-2-penem-3-carbonsäure, IR (DMSO-$d_6$): 3435; 1780; 1738; 1619 $cm^{-1}$, UV (Wasser) $\lambda_{max}$: 307 nm.
(5R,6S)-2-[(3R,S)-3-Aminobutyryloxymethyl]-6-[(1R)-1-hydroxyäthyl]-2-penem-3-carbonsäure, UV ($H_2O$) $\lambda_{max}$: 307 nm, IR (DMSO-$d_6$): 3440, 1775, 1735, 1617 $cm^{-1}$.
(5R,6S)-2-(3-Amino-3-methyl-butyryloxymethyl)-6-[(1R)-1-hydroxyäthyl[-2-penem-3-carbonsäure, UV ($H_2O$) $\lambda_{max}$: 307 nm, IR (DMSO-$d_6$): 3438, 1775, 1734, 1618 $cm^{-1}$.
(5R,6S)-2-[(2R,S)-3-Amino-2-methyl-propionyloxymethyl]-6-[(1R)-1-hydroxyäthyl]-2-penem-3-carbonsäure, UV ($H_2O$) $\lambda_{max}$: 307 nm, IR (DMSO-$d_6$): 3440, 1777, 1734, 1618 $cm^{-1}$.
(5R,6S)-2-[(3R,S)-3-Amino-3-phenyl-propionyloxymethyl]-6-[(1R)-1-hydroxyäthyl]-2-penem-3-carbonsäure, UV ($H_2O$) $\lambda_{max}$: 308 nm, IR (DMSO-$d_6$): 3439, 1774, 1734, 1617 $cm^{-1}$.
(5R,6S)-2-[(2S)-Valyloxymethyl]-6-[(1R)-1-hydroxyäthyl]-2-penem-3-carbonsäure, UV ($H_2O$) $\lambda_{max}$: 308 nm, IR (DMSO-$d_6$): 3299, 1778, 1745, 1620 $cm^{-1}$.
(5R,6S)-2-[(2R)-Valyloxymethyl]-6-[(1R)-1-hydroxyäthyl]-2-penem--3-carbonsäure, UV ($H_2O$) $\lambda_{max}$: 308 nm, IR (DMSO-$d_6$): 3427, 1778, 1745, 1621 $cm^{-1}$.
(5R,6S)-2-[(2S)-Alanyloxymethyl]-6-[(1R)-1-hydroxyäthyl]-2-penem-3-carbonsäure, UV ($H_2O$) $\lambda_{max}$: 308 nm, IR (DMSO-$d_6$): 3435, 1776, 1750, 1620 $cm^{-1}$.
(5R,6S)-2-[(2R)-Alanyloxymethyl]-6-[(1R)-1-hydroxyäthyl]-2-penem-3-carbonsäure, UV ($H_2O$) $\lambda_{max}$: 308 nm, IR (KBr): 3184, 1776, 1760, 1602 $cm^{-1}$.
(5R,6S)-2-[(4R,S)-4-Aminopentanoyloxymethyl]-6-[(1R)-1-hydroxyäthyl]-2-penem-3-carbonsäure, UV ($H_2O$) $\lambda_{max}$: 307 nm, IR (DMSO-$d_6$): 3435, 1775, 1735, 1618 $cm^{-1}$.
(5R,6S)-2-(4-Amino-4-methyl-pentanoyloxymethyl)-6-[(1R)-1-hydroxyäthyl]-2-penem-3-carbonsäure, UV ($H_2O$) $\lambda_{max}$: 307 nm, IR (DMSO-$d_6$): 3481, 1776, 1738, 1618 $cm^{-1}$.
(5R,6S)-2-[(2R,S)-4-Amino-2-methyl-butyryloxymethyl]-6-[(1R)-1-hydroxyäthyl]-2-penem-3-carbonsäure, UV ($H_2O$) $\lambda_{max}$: 307 nm, IR (DMSO-$d_6$): 3439, 1775, 1730, 1618 $cm^{-1}$.
(5R,6S)-2-(3-Amino-2,2-dimethylpropionyloxymethyl]-6-[(1R)-1-hydroxyäthyl]-2-penem-3-carbonsäure, UV ($H_2O$) $\lambda_{max}$: 307 nm, IR (DMSO-$d_6$): 3435, 1775, 1733, 1618 $cm^{-1}$.
(5R,6S)-2-(2-Amino-2-methyl-propionyloxy)-6-[(1R)-1-hydroxyäthyl]-2-penem-3-carbonsäure, UV ($H_2O$) $\lambda_{max}$: 307 nm, IR (DMSO-$d_6$): 3431, 1777, 1745, 1619 $cm^{-1}$.
(5R,6S)-2-Sarcosyloxymethyl-6-[(1R)-1-hydroxyäthyl]-2-penem-3-carbonsäure, UV ($H_2O$) $\lambda_{max}$: 307 nm, IR (DMSO-$d_6$): 3307, 1778, 1752, 1618 $cm^{-1}$.
(5R,6S)-2-(N-Methyl-N-methoxycarbonyl-glycyloxymethyl)-6-[(1R)-1-hydroxyäthyl]-2-penem-3-carbonsäure, UV ($H_2O$) $\lambda_{max}$: 307 nm, IR(DMSO-$d_6$): 3427, 1772, 1748, 1703, 1618 $cm^{-1}$.
(5R,6S)-2-[(4R,S)-Piperidin-4-ylcarbonyloxymethyl]-6-(1R)-1-hydroxyäthyl]-2-penem-3-carbonsäure, UV

($H_2O$) $\lambda_{max}$: 306 nm, IR (DMSO-$d_6$): 3435, 1774, 1731, 1618 $cm^{-1}$.
(5R,6S)-2-[(3R,S)-Piperidin-3-ylcarbonyloxymethyl]-6-[(1R)-1-hydroxyäthyl]-2-penem-3-carbonsäure, UV ($H_2O$) $\lambda_{max}$: 306 nm, IR (DMSO-$d_6$): 3439, 1775, 1730, 1618 $cm^{-1}$.

Beispiel 18: Trockenampullen oder Vials, enthaltend 0,5 g (5R,6S)-2-(4-Aminobutyryloxymethyl)-6-[(1R)-1-hydroxyäthyl]-2-penem-3-carbonsäure als Wirksubstanz, werden wie folgt hergestellt:

Zusammensetzung (für 1 Ampulle oder Vial):
Wirksubstanz 0,5 g
Mannit 0,5 g

Eine sterile wässrige Lösung der Wirksubstanz und des Mannits wird unter aseptischen Bedingungen in 5 ml-Ampullen oder 5 ml-Vials der Gefriertrocknung unterworfen und die Ampullen bzw. Vials verschlossen und geprüft.

Anstelle des obengenannten Wirkstoffs kann auch dieselbe Menge eines anderen Wirkstoffs der vorangehenden Beispiele verwendet werden.

**Patentansprüche**

1. Verbindungen der Formel

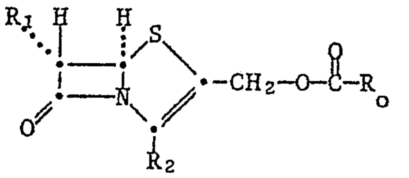

(I),

worin $R_1$ durch Hydroxy oder geschütztes Hydroxy substituiertes Niederalkyl ist, $R_2$ Carboxyl oder funktionell abgewandeltes Carboxyl bedeutet, $R_o$ Azacycloalkyl oder eine Gruppe der Formel -Y-N($R_3$,$R_4$) ist, worin $R_3$ Wasserstoff oder Niederalkyl ist, $R_4$ Wasserstoff, Niederalkyl oder Acyl ist und Y Niederalkylen, durch Phenyl substituiertes Niederalkylen oder ein Rest der Formel -$CR_5R_6$- ist, worin $R_5$ für einen über ein Kohlenstoffatom gebundenen organischen Rest steht oder zusammen mit $R_3$ gegebenenfalls durch Oxo oder Hydroxy substituiertes Niederalkylen bedeutet, und $R_6$ Wasserstoff oder Niederalkyl bedeutet, die reinen optischen Isomere von Verbindungen der Formel (I), Mischungen dieser optischen Isomere, und Salze davon.

2. Verbindungen der Formel

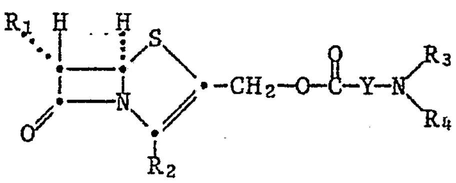

(I'),

worin $R_1$ durch Hydroxy oder geschütztes Hydroxy substituiertes Niederalkyl ist, $R_2$ Carboxyl oder funktionell abgewandeltes Carboxyl bedeutet, $R_3$ Wasserstoff oder Niederalkyl ist, $R_4$ Wasserstoff, Niederalkyl oder Acyl ist und Y geradkettiges Niederalkylen oder eine Gruppe der Formel -(CH-$R_5$)- ist, worin $R_5$ für einen über ein Kohlenstoffatom gebundenen organischen Rest steht oder zusammen mit $R_3$ gegebenenfalls durch Oxo oder Hydroxy substituiertes Nieder alkylen bedeutet, die reinen optischen Isomere von Verbindungen der Formel (I'), Mischungen dieser optischen Isomere, und Salze davon.

3. Verbindungen der Formel I gemäss Anspruch 1, worin $R_1$ durch Hydroxy oder geschütztes Hydroxy substituiertes Niederalkyl ist; $R_2$ Carboxyl, unter physiologischen Bedingungen spaltbares verestertes Carboxyl oder geschütztes Carboxyl $R_2'$ bedeutet; $R_o$ Azacycloalkyl oder eine Gruppe der Formel -Y-N($R_3$,$R_4$) ist, worin $R_3$ Wasserstoff oder Niederalkyl ist; $R_4$ Wasserstoff, Niederalkyl, Niederalkanoyl, durch Hydroxy, Niederalkoxy, Amino, Halogen, Carboxy und/oder Cyano substituiertes Niederalkanoyl; Benzoyl, durch Amino, Niederalkylamino, Diniederalkylamino, Niederalkanoylamino, Carboxy, Halogen, Nitro, Cyano und/oder Niederalkyl substituiertes Benzoyl, Phenylniederalkanoyl, im Niederalkanoylteil durch Amino oder Hydroxy substituiertes Phenylniederalkanoyl, Carbamoyl, Sulfo oder eine Aminoschutzgruppe ist, und Y geradkettiges Niederalkylen mit 1 bis 12 Kohlenstoffatomen, verzweigtkettiges Niederalkylen mit 2 bis 10 Kohlenstoffatomen, durch Phenyl substituiertes Niederalkylen oder eine Gruppe der Formel -$CR_5R_6$- ist, worin $R_5$ Niederalkyl, Niederalkenyl, Cycloalkyl, Cycloalkenyl, Cycloalkadienyl, Phenyl, Phenylniederalkyl, einen über ein Ringkohlenstoffatom gebundenen monocylischen 5- oder 6-gliedrigen gegebenenfalls partiell gesättigten Heteroarylrest mit 1-4 Ringstickstoffatomen und gegebenenfalls einem zusätzlichen Ringheteroatom ausgewählt aus der Gruppe Sauerstoff und

Schwefel, oder ein gegebenenfalls partiell gesättigtes Benzo-, Pyrido- oder Pyrimido-Derivat eines solchen 5- oder 6-gliedrigen Restes, oder einen durch einen über ein Ringkohlenstoffatom gebundenen monocyclischen 5- oder 6-gliedrigen gegebenenfalls partiell gesättigten Heteroarylrest mit 1-4 Ringstickstoffatomen und gegebenenfalls einem zusätzlichen Ringheteroatom ausgewählt aus der Gruppe Sauerstoff und Schwefel, oder einen durch einen über ein Ringstickstoffatom gebundenen monocyclischen, 5- oder 6-gliedrigen gegebenenfalls partiell gesättigten Heteroarylrest mit 1-4 Ringstickoffatomen substituierten Niederalkylrest darstellt, welche Reste $R_5$ unsubstituiert oder durch Hydroxy, Niederalkoxy, Niederalkanoyloxy, Halogen, Mercapto, Niederalkylthio, gegebenenfalls durch Hydroxy, Niederalkoxy, Carboxy, Niederalkoxycarbonyl, Carbamoyl, gegebenenfalls N-niederalkyliertes Amino, Niederalkylenamino, Niederalkanoylamino, gegebenenfalls durch Amino und/oder Carboxy substituiertes Niederalkoxycarbonylamino, Mercapto, Niederalkylthio oder Sulfo substituiertes Niederalkyl; Amino, Niederalkylamino, Diniederalkylamino, Niederalkylenamino, Guanidino, Ureido, Niederalkanoylamino, gegebenenfalls durch Amino und/oder Carboxy substituiertes Niederalkoxycarbonylamino, Carboxyl, Niederalkoxycarbonyl, Carbamoyl, N-mono- oder N,N-diniederalkyliertes Carbamoyl, Cyano, Sulfo, Sulfamoyl, gegebenenfalls durch Niederalkyl, Nitro, Amino, Hydroxy, Niederalkoxy, Carboxy und/oder Halogen substituiertes Phenyl, Nitro, Oxo und/oder Oxido substituiert sind; oder worin $R_5$ zusammen mit $R_3$ gegebenenfalls durch Hydroxy oder Oxo substituiertes Niederalkylen bedeutet, und $R_6$ Wasserstoff oder Niederalkyl bedeutet, die reinen optischen Isomere von Verbindungen der Formel I, welche im Rest $R_1$ und/oder $R_o$Chiralitätszentren besitzen, Mischungen dieser optischen Isomere und Salze von solchen Verbindungen der Formel I, die eine salzbildende Gruppe aufweisen.

4. Verbindungen der Formel I′ gemäss Anspruch 2, worin $R_1$, $R_2$, $R_3$ und $R_4$ die in Anspruch 3 angegebenen Bedeutungen haben und Y geradkettiges Niederalkylen mit 1 bis 7 Kohlenstoffatomen oder eine Gruppe der Formel $-(CHR_5)-$ ist, worin $R_5$ die in Anspruch 3 angegebenen Bedeutungen hat, die reinen optischen Isomere von Verbindungen der Formel I′, welche im Rest $R_1$ und/oder Y Chiralitätszentren besitzen, Mischungen dieser optischen Isomere und Salze von solchen Verbindungen der Formel I′, die eine salzbildende Gruppe aufweisen.

5. Verbindungen der Formel I gemäss Anspruch 1, worin $R_1$ in α-Stellung durch Hydroxy, Triniederalkylsilyloxy, Niederalkenyloxyoxalyloxy oder Niederalkenyloxycarbonyloxy substituiertes Niederalkyl bedeutet, $R_2$ Carboxyl, 4-Nitrobenzyloxycarbonyl, Niederalkenyloxycarbonyl, in 2-Stellung durch Triniederalkylsilyl substituiertes Aethoxycarbonyl oder eine unter physiologischen Bedingungen spaltbare veresterte Carboxylgruppe ist und $R_o$ für den Acylrest einer genetisch enkodierten Aminosäure, oder für den Acylrest von β-Alanin, Homoserin, 2-, 3- oder 4-Aminobuttersäure, 2,3-Diaminopropionsäure, 2,3-Diaminobuttersäure, 5-Aminopentansäure, 6-Aminohexansäure, 8-Aminooctansäure, 11-Aminoundecansäure, α- oder β-Aminoisobuttersäure, 3-Amino-3-methyl-buttersäure, 4-Amino-4-methyl-pentansäure, 4-Amino-1-methyl-buttersäure, Aminopivalinsäure, 4-Aminopentansäure, 3-Amino-3-phenyl-propionsäure, Citrullin, Ornithin, Piperidin-3-yl-carbonsäure, Piperidin-4-ylcarbonsäure, 3-Hydroxyprolin oder Pyrrolidin-5-on-2-carbonsäure oder für ein N-Niederalkyl-Derivat eines solchen Acylrestes steht, die reinen Diastereomere von Verbindungen der Formel (I), die im Rest $R_1$ und/oder $R_o$ Chiralitätszentren besitzen, Mischungen solcher Diastereomere, und Salze von solchen Verbindungen der Formel (I), die eine salzbildende Gruppe aufweisen.

6. Verbindungen der Formel I′ gemäss Anspruch 2, worin $R_1$ und $R_2$ die in Anspruch 5 angegebenen Bedeutungen haben und der Rest $-C(=O)-Y-N(R_3,R_4)$ für den Acylrest einer genetisch enkodierten Aminosäure, für β-Alanyl, 2- oder 4-Aminobutyryl, 2,3-Diaminobutyryl, 2,3-Diaminopropionyl, Citrullyl, Ornithyl, 3-Hydroxyprolyl, Pyrrolidin-5-on-2-carbonyl oder für ein N-Niederalkyl-Derivat eines solchen Acylrestes steht, die reinen optischen Isomere von Verbindungen der Formel I′, welche im Rest $R_1$ und/oder Y Chiralitätszentren besitzen, Mischungen dieser optischen Isomere und Salze von solchen Verbindungen der Formel I′, die eine salzbildende Gruppe aufweisen.

7. Verbindungen der Formel I gemäss Anspruch 1, worin $R_1$ Hydroxymethyl oder 1-Hydroxyäthyl ist, $R_2$ Carboxyl oder unter physiologischen Bedingungen spaltbares verestertes Carboxyl bedeutet und $R_o$ für Glycyl, Alanyl, Lysyl, β-Alanyl, 2,3-Diaminopropionyl, 3-Amino-3-methyl-butyryl, 4-Aminobutyryl oder für ein N-Methyl-Derivat eines solchen Aminoacyl-Restes steht, die reinen Diastereomere von Verbindungen der Formel (I), worin $R_1$ 1-Hydroxyäthyl ist und/oder die im Rest $R_o$ ein Chiralitätszentrum besitzen, Mischungen solcher Diastereomere, und Salze von solchen Verbindungen der Formel (I), die eine salzbildende Gruppe aufweisen.

8. Verbindungen der Formel I′ gemäss Anspruch 2, worin $R_1$ und $R_2$ die in Anspruch 7 angegebenen Bedeutungen haben und der Rest $-C(=O)-Y-N(R_3,R_4)$ für Glycyl, Alanyl, Lysyl, β-Alanyl, 2,3-Diaminopropionyl oder für ein N-Methyl-Derivat eines solchen Aminoacyl-Restes steht, die reinen Diastereomere von Verbindungen der Formel I′, worin $R_1$ 1-Hydroxyäthyl ist und/oder die im Rest Y ein Chiralitätszentrum besitzen, Mischungen solcher Diastereomere, und Salze von solchen Verbindungen der Formel I′, die eine salzbildende Gruppe aufweisen.

9. Verbindungen der Formel I gemäss Anspruch 1, worin $R_1$ 1-Hydroxyäthyl ist, $R_2$ Carboxyl oder unter physiologischen Bedingungen spaltbares verestertes Carboxyl bedeutet und $R_o$ für β-Alanyl, 3-Amino-3-methyl-butyryl oder 4-Aminobutyryl steht, und Salze davon.

10. (5R,6S)-2-Glycyloxymethyl-6-[(1R)-1-hydroxyäthyl]-2-penem-3-carbonsäure gemäss Anspruch 2.

11. (5R,6S)-2-[(1S)-Lysyloxymethyl]-6-[(1R)-1-hydroxyäthyl]-2-penem-3-carbonsäure gemäss Anspruch 2.

12. (5R,6S)-2-(3-Aminopropionyloxymethyl)-6-[(1R)-1-hydroxyäthyl]-2-penem-3-carbonsäure gemäss Anspruch 2.

13. (5R,6S)-2-[(2R,S)-2,3-Diaminopropionyloxymethyl]-6-[(1R)-1-hydroxyäthyl]-2-penem-3-carbonsäure gemäss Anspruch 2.

14. (5R,6S)-2-[(2S)-Prolyloxymethyl]-6-(1R)-1-hydroxyäthyl]-2-penem-3-carbonsäure gemäss Anspruch 2.

15. (5R,6S)-2-(4-Aminobutyryloxymethyl)-6-[(1R)-1-hydroxyäthyl]-2-penem-3-carbonsäure gemäss Anspruch 1.

16. (5R,6S)-2-(3-Amino-3-methyl-butyryloxymethyl)-6-[(1R)-1-hydroxyäthyl]-2-penem-3-carbonsäure gemäss Anspruch 1.

17. Eine Verbindung der Formel I gemäss Anspruch 1 zur Anwendung in einem Verfahren zur therapeutischen Behandlung des menschlichen und tierischen Körpers.

18. Eine Verbindung der Formel I' gemäss Anspruch 2 zur Anwendung in einem Verfahren zur therapeutischen Behandlung des menschlichen und tierischen Körpers.

19. Pharmazeutisches Präparat enthaltend eine Verbindung der Formel I gemäss Anspruch 1.

20. Pharmazeutisches Präparat enthaltend eine Verbindung der Formel I' gemäss Anspruch 2.

21. Verwendung von Verbindungen der Formel I gemäss Anspruch 1 zur Herstellung von pharmazeutischen Präparaten.

22. Verwendung von Verbindungen der Formel I' gemäss Anspruch 2 zur Herstellung von pharmazeutischen Präparaten.

23. Verbindungen der Formel I gemäss Anspruch 1 als antibiotische Mittel.

24. Verbindungen der Formel I' gemäss Anspruch 2 als antibiotische Mittel.

25. Verfahren zur Herstellung von Verbindungen der Formel I gemäss Anspruch 1, dadurch gekennzeichnet, dass man

a) in einer Verbindung der Formel

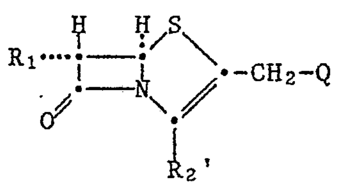

(II),

worin $R_1$ die unter Formel (I) angegebene Bedeutung hat, $R_2'$ eine geschützte Carboxylgruppe ist und Q eine in $R_o$-C(=O)-O- überführbare Gruppe ist, die Gruppe Q in $R_o$-C(=O)-O- überführt, oder

b) eine Ylid-Verbindung der Formel

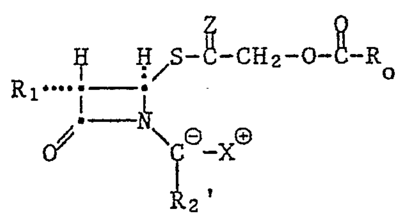

(III),

worin $R_1$ und $R_o$ die unter Formel I angegebenen Bedeutungen haben, $R_2'$ eine geschützte Carboxylgruppe ist, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

c) eine Verbindung der Formel

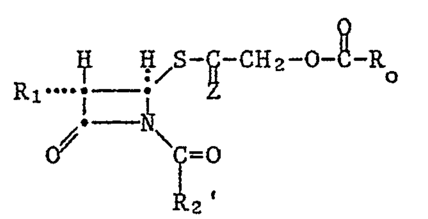

(IV),

worin $R_1$ $R_o$ die unter Formel I angegebenen Bedeutungen haben, Z Sauerstoff oder Schwefel ist und $R_2'$ eine geschützte Carboxylgruppe ist, mit einer organischen Verbindung des dreiwertigen Phosphors behandelt, und wenn erwünscht oder notwendig, in einer erhältlichen Verbindung der

Formel I eine geschützte funktionelle Gruppen in die freie funktionelle Gruppe überführt, und/oder, wenn erwünscht, in einer erhältlichen Verbindung der Formel I eine freie Carboxylgruppe $R_2$ in eine unter physiologischen Bedingungen spaltbare veresterte Carboxylgruppe überführt, und/oder, wenn erwünscht, in einer erhältlichen Verbindung der Formel I einen Rest $R_o$ in einen anderen Rest $R_o$ überführt, und/oder, wenn erwünscht, ein erhältliches Gemisch von isomeren Verbindungen der Formel I in die einzelnen Isomere auftrennt, und/oder, wenn erwünscht, eine erhältliche Verbindung mit salzbildender Gruppe in ein Salz oder ein erhältliches Salz in die freie Verbindung oder in ein anderes Salz überführt.

26. Verfahren zur Herstellung von Verbindungen der Formel I′ gemäss Anspruch 2, dadurch gekennzeichnet, dass man

a) in einer Verbindung der Formel

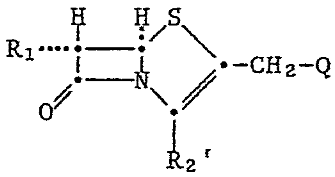

(II),

worin $R_1$ die unter Formel (I′) angegebene Bedeutung hat, $R_2'$ eine geschützte Carboxylgruppe ist und Q eine in den Rest

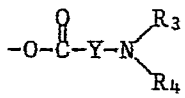

(IIa),

worin Y, $R_3$ und $R_4$ die unter Formel (I′) angegebenen Bedeutungen haben, überführbare Gruppe ist, die Gruppe Q in den Rest

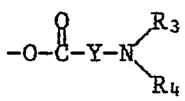

(IIa)

überführt, oder

b) eine Ylid-Verbindung der Formel

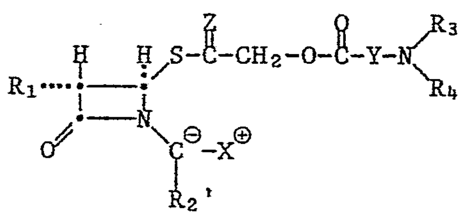

(III′),

worin $R_1$, $R_3$, $R_4$, und Y die unter Formel I′ angegebenen Bedeutungen haben, $R_2'$ eine geschützte Carboxylgruppe ist, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

c) eine Verbindung der Formel

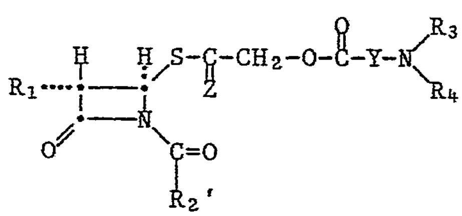

(IV′),

worin $R_1$, $R_3$, $R_4$, und Y die unter Formel I′ angegebenen Bedeutungen haben, Z Sauerstoff oder Schwefel ist und $R_2'$ eine geschützte Carboxylgruppe ist, mit einer organischen Verbindung des dreiwertigen Phosphors behandelt, und wenn erwünscht oder notwendig, in einer erhältlichen

Verbindung der Formel I' eine geschützte funktionelle Gruppe in die freie funktionelle Gruppe überführt, und/oder, wenn erwünscht, in einer erhältlichen Verbindung der Formel I' eine freie Carboxylgruppe $R_2$ in eine unter physiologischen Bedingungen spaltbare veresterte Carboxylgruppe überführt, und/oder, wenn erwünscht, in einer erhältlichen Verbindung der Formel I' einen Rest $R_3$ und/oder $R_4$ und/oder Y in einen anderen Rest $R_3$ und/oder $R_4$ und/oder Y überführt, und/oder, wenn erwünscht, ein erhältliches Gemisch von isomeren Verbindungen der Formel I' in die einzelnen Isomere auftrennt, und/oder, wenn erwünscht, eine erhältliche Verbindung mit salzbildender Gruppe in ein Salz oder ein erhältliches Salz in die freie Verbindung oder in ein anderes Salz überführt.

27. Die nach dem Verfahren gemäss Anspruch 25 erhältlichen Verbindungen.

28. Die nach dem Verfahren gemäss Anspruch 26 erhältlichen Verbindungen.

Patentansprüche für folgende Vertragstaaten: AT, GR, ES

1. Verfahren zur Herstellung von Verbindungen der Formel

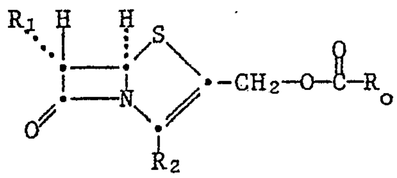

(I),

worin $R_1$ durch Hydroxy oder geschütztes Hydroxy substituiertes Niederalkyl ist, $R_2$ Carboxyl oder funktionell abgewandeltes Carboxyl bedeutet, $R_o$ Azacycloalkyl oder eine Gruppe der Formel -Y-N($R_3$,$R_4$) ist, worin $R_3$ Wasserstoff oder Niederalkyl ist, $R_4$ Wasserstoff, Niederalkyl oder Acyl ist und Y Niederalkylen, durch Phenyl substituiertes Niederalkylen oder ein Rest der Formel -$CR_5R_6$- ist, worin $R_5$ für einen über ein Kohlenstoffatom gebundenen organischen Rest steht oder zusammen mit $R_3$ gegebenenfalls durch Oxo oder Hydroxy substituiertes Niederalkylen bedeutet, und $R_6$ Wasserstoff oder Niederalkyl bedeutet, die reinen optischen Isomere von Verbindungen der Formel (I), Mischungen dieser optischen Isomere und Salzen davon, dadurch gekennzeichnet, dass man

a) in einer Verbindung der Formel

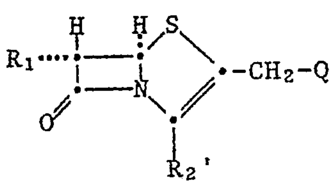

(II),

worin $R_1$ die unter Formel (I) angegebene Bedeutung hat, $R_2'$ eine geschützte Carboxylgruppe ist und Q eine in $R_o$-C(=O)-O- überführbare Gruppe ist, die Gruppe Q in $R_o$-C(=O)-O- überführt, oder

b) eine Ylid-Verbindung der Formel

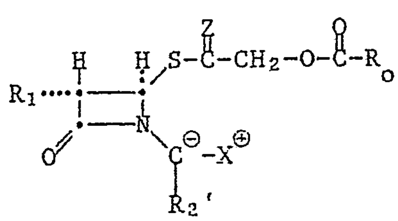

(III),

worin $R_1$ und $R_o$ die unter Formel I angegebenen Bedeutungen haben, $R_2'$ eine geschützte Carboxylgruppe ist, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

c) eine Verbindung der Formel

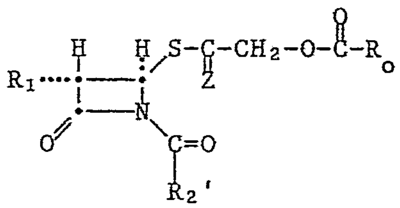 (IV),

worin $R_1$ und $R_o$ die unter Formel I angegebenen Bedeutungen haben, Z Sauerstoff oder Schwefel ist und $R_2'$ eine geschützte Carboxylgruppe ist, mit einer organischen Verbindung des dreiwertigen Phosphors behandelt, und wenn erwünscht oder notwendig, in einer erhältlichen Verbindung der Formel I eine geschützte funktionelle Gruppen in die freie funktionelle Gruppe überführt, und/oder, wenn erwünscht, in einer erhältlichen Verbindung der Formel I eine freie Carboxylgruppe $R_2$ in eine unter physiologischen Bedingungen spaltbare veresterte Carboxylgruppe überführt, und/oder, wenn erwünscht, in einer erhältlichen Verbindung der Formel I einen Rest $R_o$ in einen anderen Rest $R_o$ überführt, und/oder, wenn erwünscht, ein erhältliches Gemisch von isomeren Verbindungen der Formel I in die einzelnen Isomere auftrennt, und/oder, wenn erwünscht, eine erhältliche Verbindung mit salzbildender Gruppe in ein Salz oder ein erhältliches Salz in die freie Verbindung oder in ein anderes Salz überführt.

2. Verfahren zur Herstellung von Verbindungen der Formel

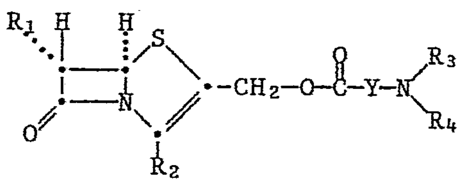 (I'),

worin $R_1$ durch Hydroxy oder geschütztes Hydroxy substituiertes Niederalkyl ist, $R_2$ Carboxyl oder funktionell abgewandeltes Carboxyl bedeutet, $R_3$ Wasserstoff oder Niederalkyl ist, $R_4$ Wasserstoff, Niederalkyl oder Acyl ist und Y geradkettiges Niederalkylen oder eine Gruppe der Formel $-(CH-R_5)-$ ist, worin $R_5$ für einen über ein Kohlenstoffatom gebundenen organischen Rest steht oder zusammen mit $R_3$ gegebenenfalls durch Oxo oder Hydroxy substituiertes Niederalkylen bedeutet, den reinen optischen Isomeren von Verbindungen der Formel I', Mischungen dieser optischen Isomere und Salzen davon, dadurch gekennzeichnet, dass man

a) in einer Verbindung der Formel

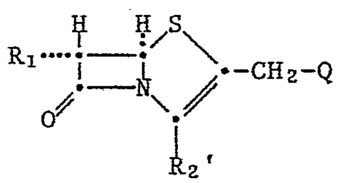 (II),

worin $R_1$ die unter Formel (I') angegebene Bedeutung hat, $R_2'$ eine geschützte Carboxylgruppe ist und Q eine in den Rest

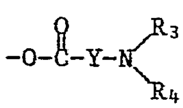 (IIa),

worin Y, $R_3$ und $R_4$ die unter Formel (I') angegebenen Bedeutungen habe, überführbare Gruppe ist, die Gruppe Q in den Rest

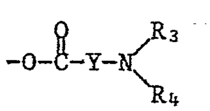 (IIa)

überführt, oder

b) eine Ylid-Verbindung der Formel

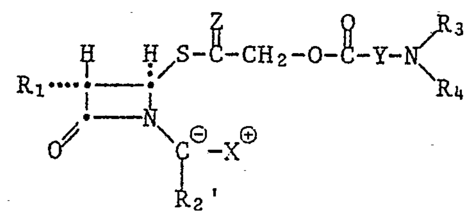

(III'),

worin $R_1$, $R_3$, $R_4$, und Y die unter Formel I' angegebenen Bedeutungen haben, $R_2'$ eine geschützte Carboxylgruppe ist, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder

c) eine Verbindung der Formel

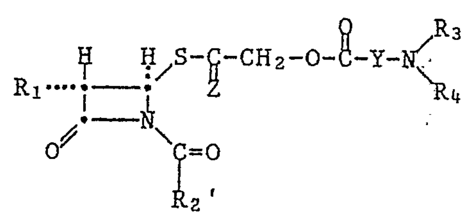

(IV'),

worin $R_1$, $R_3$, $R_4$, und Y die unter Formel I' angegebenen Bedeutungen haben, Z Sauerstoff oder Schwefel ist und $R_2'$ eine geschützte Carboxylgruppe ist, mit einer organischen Verbindung des dreiwertigen Phosphors behandelt, und wenn erwünscht oder notwendig, in einer erhältlichen Verbindung der Formel I' eine geschützte funktionelle Gruppe in die freie funktionelle Gruppe überführt, und/oder, wenn erwünscht, in einer erhältlichen Verbindung der Formel I' eine freie Carboxylgruppe $R_2$ in eine unter physiologischen Bedingungen spaltbare veresterte Carboxylgruppe überführt, und/oder, wenn erwünscht, in einer erhältlichen Verbindung der Formel I' einen Rest $R_3$ und/oder $R_4$ und/oder Y in einen anderen Rest $R_3$ und/oder $R_4$ und/oder Y überführt, und/oder, wenn erwünscht, ein erhältliches Gemisch von isomeren Verbindungen der Formel I' in die einzelnen Isomere auftrennt, und/oder, wenn erwünscht, eine erhältliche Verbindung mit salzbildender Gruppe in ein Salz oder ein erhältliches Salz in die freie Verbindung oder in ein anderes Salz überführt.

3. Verfahren zur Herstellung von Verbindungen der Formel I gemäss Anspruch 1, worin $R_1$ durch Hydroxy oder geschütztes Hydroxy substituiertes Niederalkyl ist; $R_2$ Carboxyl, unter physiologischen Bedingungen spaltbares verestertes Carboxyl oder geschütztes Carboxyl $R_2'$ bedeutet; $R_o$ Azacycloalkyl oder eine Gruppe der Formel -Y-N($R_3$,$R_4$) ist, worin $R_3$ Wasserstoff oder Niederalkyl ist; $R_4$ Wasserstoff, Niederalkyl, Niederalkanoyl, durch Hydroxy, Niederalkoxy, Amino, Halogen, Carboxy, und/oder Cyano substituiertes Niederalkanoyl; Benzoyl, durch Amino, Niederalkylamino, Diniederalkylamino, Niederalkanoylamino, Carboxy, Halogen, Nitro, Cyano und/oder Niederalkyl substituiertes Benzoyl, Phenylniederalkanoyl, im Niederalkanoylteil durch Amino oder Hydroxy substituiertes Phenylniederalkanoyl, Carbamoyl, Sulfo oder eine Aminoschutzgruppe ist, und Y geradkettiges Niederalkylen, mit 1 bis 12 Kohlenstoffatomen, verzweigtkettiges Niederalkylen mit 2 bis 10 Kohlenstoffatomen, durch Phenyl substituiertes Niederalkylen oder eine Gruppe der Formel $-CR_5R_6$ ist, worin $R_5$ Niederalkyl, Niederalkenyl, Cycloalkyl, Cycloalkenyl, Cycloalkadienyl, Phenyl, Phenylniederalkyl, einen über ein Ringkohlenstoffatom gebundenen monocyclischen 5- oder 6-gliedrigen gegebenenfalls partiell gesättigten Heteroarylrest mit 1-4 Ringstickstoffatomen und gegebenenfalls einem zusätzlichen Ringheteroatom ausgewählt aus der Gruppe Sauerstoff und Schwefel, oder ein gegebenenfalls partiell gesättigtes Benzo-, Pyrido- oder Pyrimido-Derivat eines solchen 5- oder 6-gliedrigen Restes, oder einen durch einen über ein Ringkohlenstoffatom gebundenen monocyclischen 5- oder 6-gliedrigen gegebenenfalls partiell gesättigten Heteroarylrest mit 1-4 Ringstickstoffatomen und gegebenenfalls einem zusätzlichen Ringheteroatom ausgewählt aus der Gruppe Sauerstoff und Schwefel, oder einen durch einen über ein Ringstickstoffatom gebundenen monocyclischen, 5- oder 6-gliedrigen gegebenenfalls partiell gesättigten Heteroarylrest mit 1-4 Ringstickoffatomen substituierten Niederalkylrest darstellt, welche Reste $R_5$ unsubstituiert oder durch Hydroxy, Niederalkoxy, Niederalkanoyloxy, Halogen, Mercapto, Niederalkylthio, gegebenenfalls durch Hydroxy, Niederalkoxy, Carboxy, Niederalkoxycarbonyl, Carbamoyl, gegebenenfalls N-niederalkyliertes Amino, Niederalkylenamino, Niederalkanoylamino, gegebenenfalls durch Amino und/oder Carboxy substituiertes Niederalkoxycarbonylamino, Mercapto, Niederalkylthio oder Sulfo substituiertes Niederalkyl; Amino, Niederalkylamino, Diniederalkylamino, Niederalkylenamino, Guanidino, Ureido, Niederalkanoylamino, gegebenenfalls durch Amino und/oder Carboxy substituiertes Niederalkoxycarbonylamino, Carboxyl, Niederalkoxycarbonyl, Carbamoyl, N-mono-oder N,N-diniederalkyliertes Carbamoyl, Cyano, Sulfo, Sulfamoyl, gegebenenfalls durch Niederalkyl, Nitro, Amino, Hydroxy, Niederalkoxy, Carboxy

und/oder Halogen substituiertes Phenyl, Nitro, Oxo und/oder Oxido substituiert sind; oder worin $R_5$ zusammen mit $R_3$ gegebenenfalls durch Hydroxy oder Oxo substituiertes Niederalkylen bedeutet, und $R_6$ Wasserstoff oder Niederalkyl bedeutet, den reinen optischen Isomeren von Verbindungen der Formel I, welche im Rest $R_1$ und/oder $R_o$ Chiralitätszentren besitzen, Mischungen dieser optischen Isomeren und Salzen von solchen Verbindungen der Formel I, die eine salzbildende Gruppe aufweisen.

4. Verfahren zur Herstellung von Verbindungen der Formel I' gemäss Anspruch 2, worin $R_1$, $R_2$, $R_3$ und $R_4$ die in Anspruch 3 angegebenen Bedeutungen haben und Y geradkettiges Niederalkylen mit 1 bis 7 Kohlenstoffatomen oder eine Gruppe der Formel $-(CHR_5)-$ ist, worin $R_5$ die in Anspruch 3 angegebenen Bedeutungen hat, den reinen optischen Isomeren von Verbindungen der Formel I', welche im Rest $R_1$, und/oder Y Chiralitätszentren besitsen, Mischungen dieser optischen Isomere und Salzen von solchen Verbindungen der Formel I', die eine salzbildende Gruppe aufweisen.

5. Verfahren zur Herstellung von Verbindungen der Formel I gemäss Anspruch 1, worin $R_1$ in α-Stellung durch Hydroxy, Triniederalkylsilyloxy, Niederalkenyloxyoxalyloxy oder Niederalkenyloxycarbonyloxy substituiertes Niederalkyl bedeutet, $R_2$ Carboxyl, 4-Nitrobenzyloxycarbonyl, Niederalkenyloxycarbonyl, in 2-Stellung durch Triniederalkylsilyl substituiertes Aethoxycarbonyl oder eine unter physiologischen Bedingungen spaltbare veresterte Carboxylgruppe ist und $R_o$ für den Acylrest einer genetisch enkodierten Aminosäure, oder für den Acylrest von β-Alanin, Homoserin, 2-, 3- oder 4-Aminobuttersäure, 2,3-Diaminopropionsäure, 2,3-Diaminobuttersäure, 5-Aminopentansäure, 6-Aminohexansäure, 8-Aminooctansäure, 11-Aminoundecansäure, α- oder β-Aminoisobuttersäure, 3-Amino-3-methyl-buttersäure, 4-Amino-4-methyl-pentansäure, 4-Amino-1-methyl-buttersäure, Aminopivalinsäure, 4-Aminopentansäure, 3-Amino-3-phenyl-propionsäure, Citrullin, Ornithin, Piperidin-3-yl-carbonsäure, Piperidin-4-yl-carbonsäure, 3-Hydroxyprolin oder Pyrrolidin-5-on-2-carbonsäure oder für ein N-Niederalkyl-Derivat eines solchen Acylrestes steht, den reinen optischen Isomeren von Verbindungen der Formel I, welche im Rest $R_1$ und/oder $R_o$ Chiralitätszentren besitzen, Mischungen dieser optischen Isomeren und Salzen von solchen Verbindungen der Formel I, die eine salzbildende Gruppe aufweisen.

6. Verfahren zur Herstellung von Verbindungen der Formel I' gemäss Anspruch 2, worin $R_1$ und $R_2$ die in Anspruch 5 angegebenen Bedeutungen haben und der Rest $-C(=O)-Y-N(R_3,R_4)$ für den Acylrest einer genetisch enkodierten Aminosäure, für β-Alanyl, 2- oder 4-Aminobutyryl, 2,3-Diaminobutyryl, 2,3-Diaminopropionyl, Citrullyl, Ornithyl, 3-Hydroxyprolyl, Pyrrolidin-5-on-2-carbonyl oder für ein N-Niederalkyl-Derivat eines solchen Acylrestes steht, den reinen optischen Isomeren von Verbindungen der Formel I', welche im Rest $R_1$ und/oder Y Chiralitätszentren besitzen, Mischungen dieser optischen Isomere und Salzen von solchen Verbindungen der Formel I', die eine salzbildende Gruppe aufweisen.

7. Verfahren zur Herstellung von Verbindungen der Formel I gemäss Anspruch 1, worin $R_1$ Hydroxymethyl oder 1-Hydroxyäthyl ist, $R_2$ Carboxyl oder unter physiologischen Bedingungen spaltbares verestertes Carboxyl bedeutet und $R_o$ für Glycyl, Alanyl, Lysyl, β-Alanyl, 2,3-Diaminopropionyl, 3-Amino-3-methyl-butyryl, 4-Aminobutyryl oder für ein N-Methyl-Derivat eines solchen Aminoacyl-Restes steht, den reinen Diastereomeren von Verbindungen der Formel I, worin $R_1$ 1-Hydroxyäthyl ist und/oder die im Rest $R_o$ ein Chiralitätszentrum besitzen, Mischungen solcher Diastereomere, und Salzen von solchen Verbindungen der Formel I, die eine salzbildende Gruppe aufweisen.

8. Verfahren zur Herstellung von Verbindungen der Formel I' gemäss Anspruch 2, worin $R_1$ und $R_2$ die in Anspruch 7 angegebenen Bedeutungen haben und der Rest $-C(=O)-Y-N(R_3,R_4)$ für Glycyl, Alanyl, Lysyl, β-Alanyl, 2,3-Diaminopropionyl oder für ein N-Methyl-Derivat eines solchen Aminoacyl-Restes steht, den reinen Diastereomeren von Verbindungen der Formel I', worin $R_1$ 1-Hydroxyäthyl ist und/oder die im Rest Y ein Chiralitätszentrum besitzen, Mischungen solcher Diastereomere, und Salzen von solchen Verbindungen der Formel I', die eine salzbildende Gruppe aufweisen.

9. Verfahren zur Herstellung von Verbindungen der Formel I gemäss Anspruch 1, worin $R_1$ 1-Hydroxyäthyl ist, $R_2$ Carboxyl oder unter physiologischen Bedingungen spaltbares verestertes Carboxyl bedeutet und $R_o$ für β-Alanyl, 3-Amino-3-methyl-butyryl oder 4-Aminobutyryl steht, und Salzen davon.

10. Verfahren gemäss Anspruch 2 zur Herstellung von (5R,6S)-2-Glycyloxymethyl-6-[(1R)-1-hydroxyäthyl]-2-penem-3-carbonsäure.

11. Verfahren gemäss Anspruch 2 zur Herstellung von (5R,6S)-2-[(1S)-Lysyloxymethyl]-6-[(1R)-1-hydroxyäthyl]-2-penem-3-carbonsäure.

12. Verfahren gemäss Anspruch 2 zur Herstellung von (5R,6S)-2-(3-Aminopropionyloxymethyl)-6-[(1R)-1-hydroxyäthyl]-2-penem-3-carbonsäure.

13. Verfahren gemäss Anspruch 2 zur Herstellung von (5R,6S)-2-[(2R,S)-2,3-Diaminopropionyloxymethyl]-6-[(1R)-1-hydroxyäthyl]-2-penem-3-carbonsäure.

14. Verfahren gemäss Anspruch 2 zur Herstellung von (5R,6S)-2-[(2S)-Prolyloxymethyl]-6-[(1R)-1-hydroxyäthyl]-2-penem-3-carbonsäure.

15. Verfahren gemäss Anspruch 1 zur Herstellung von (5R,6S)-2-(4-Aminobutyryloxymethyl)-6-[(1R)-1-hydroxyäthyl]-2-penem-3-carbonsäure.

16. Verfahren gemäss Anspruch 1 zur Herstellung von (5R,6S)-2-(3-Amino-3-methyl-butyryloxymethyl)-6-[(1R)-1-hydroxyäthyl]-2-penem-3-carbonsäure.

17. Verfahren zur Herstellung von pharmazeutischen Präparaten, dadurch gekennzeichnet, dass man eine nach dem Verfahren gemäss Anspruch 1 erhältliche Verbindung der Formel I oder ein

pharmazeutisch verwendbares Salz einer solchen Verbindung mit einem pharmazeutisch verwendbaren Trägermaterial mischt.

18. Verfahren zur Herstellung von pharmazeutischen Präparaten, dadurch gekennzeichnet, dass man eine nach dem Verfahren gemäss Anspruch 2 erhätliche Verbindung der Formel I' oder ein pharmazeutisch verwendbares Salz einer solchen Verbindung mit einem pharmazeutisch verwendbaren Trägermaterial mischt.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 87 81 0081

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP - A - 0 070 204 (SUMITOMO)<br>* Seiten 70-73; Beispiele 54,56; Ansprüche * | 1,19,25,<br>26 | C 07 D 499/00<br>A 61 K 31/43//<br>C 07 D 205/08<br>C 07 F 9/65 |
| A | FR - A - 2 550 533 (FARMITALIA CARLO ERBA)<br>* Patentansprüche * | 1,25,<br>26 | |
| P,X | EP - A - 0 201 206 (FARMITALIA CARLO ERBA)<br>* Spalten 11,12, Formel; Spalten 21,22, Verbindung 41; Ansprüche * | 1,19,<br>25,26 | |

RECHERCHIERTE SACHGEBIETE (Int. Cl.4)

C 07 D 499/00
A 61 K 31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-16,19-28
Unvollständig recherchierte Patentansprüche: 17,18
Nicht recherchierte Patentansprüche:
Grund für die Beschränkung der Recherche:

Verfahren zur chirurgischen oder therapeutischen Behandlung des menschlichen oder tierischen Körpers (Siehe Art. 52(4) des Europäischen Patentübereinkommens).

| Recherchenort | Abschlußdatum der Recherche | Prüfer |
|---|---|---|
| Den Haag | 06-04-1987 | CHOULY |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1505.1 03.82